(19) Europäisches Patentamt / European Patent Office / Office européen des brevets

(11) EP 4 424 819 A1

(12) EUROPEAN PATENT APPLICATION
published in accordance with Art. 153(4) EPC

(43) Date of publication:
04.09.2024 Bulletin 2024/36

(21) Application number: 22887108.3

(22) Date of filing: 27.10.2022

(51) International Patent Classification (IPC):
C12N 5/077 (2010.01)          A61L 27/38 (2006.01)
A61P 9/04 (2006.01)           C12N 5/0735 (2010.01)
C12Q 1/02 (2006.01)

(52) Cooperative Patent Classification (CPC):
A61L 27/38; A61P 9/04; C12N 5/06; C12Q 1/02

(86) International application number:
PCT/JP2022/040098

(87) International publication number:
WO 2023/074783 (04.05.2023 Gazette 2023/18)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.10.2021 JP 2021176040

(71) Applicants:
• FUJIFILM Corporation
Tokyo 106-8620 (JP)
• OSAKA UNIVERSITY
Suita-shi
Osaka 565-0871 (JP)

(72) Inventors:
• MIYOSHI Hayato
Ashigarakami-gun, Kanagawa 258-8577 (JP)
• LIU, Li
Suita-shi, Osaka 565-0871 (JP)
• SAWA, Yoshiki
Suita-shi, Osaka 565-0871 (JP)
• LI, Junjun
Suita-shi, Osaka 565-0871 (JP)
• MIYAGAWA, Shigeru
Suita-shi, Osaka 565-0871 (JP)
• TAKEDA, Maki
Suita-shi, Osaka 565-0871 (JP)

(74) Representative: Hoffmann Eitle
Patent- und Rechtsanwälte PartmbB
Arabellastraße 30
81925 München (DE)

(54) METHOD FOR PRODUCING MYOCARDIAL CELL LAYER, MYOCARDIAL CELL LAYER, AND USE THEREOF

(57)    An object of the present invention is to provide a production method of a myocardial cell layer, and a myocardial cell layer, in which myocardial cells can be matured without using a complicated control device and can be maintained for a long period of time, and to provide a kit for evaluating a pharmaceutical candidate substance, a transplantation material, and an evaluation method of a pharmaceutical candidate substance. According to the present invention, there is provided a production method of a myocardial cell layer including the following steps (1) to (3).

(1) seeding a myocardial cell induced from a pluripotent stem cell in a single layer on a seeding surface having a shape shown in the following (1-1) to form a myocardial cell layer,

(1-1) in a case where spread of the seeding surface in a horizontal direction is a plane figure, a shape thereof is a shape obtained by hollowing out at least a part of a first plane figure of an optional shape having uniform spread in a second plane figure of another optional shape having uniform spread without contacting an outer periphery of the first plane figure of the optional shape, where an average value of a length of the outer periphery of the first plane figure and a length of an outer periphery of the second plane figure satisfies the following formula;

$$x \geq v/10$$

in the formula, x represents the average value of the length of the outer periphery of the first plane figure and the length of the outer periphery of the second plane figure, where a unit thereof is cm, and v represents a conduction velocity of the myocardial cell induced from the pluripotent stem cell, where a unit thereof is $cm \cdot s^{-1}$,

(Cont. next page)

EP 4 424 819 A1

(2) circulating and proceeding an electrical signal or a calcium signal in the myocardial cell layer, and
(3) culturing the cells for 8 days or more while maintaining a state of the (2).

## Description

### Technical Field

The present invention relates to a production method of a mature myocardial cell layer, which can be maintained for a long period of time. The present invention further relates to a mature myocardial cell layer which can be maintained for a long period of time. The present invention further relates to a kit for evaluating a pharmaceutical candidate substance, a transplantation material, and an evaluation method of a pharmaceutical candidate substance, using the myocardial cell layer described above.

### Background Art

In recent years, a method of inducing differentiation of a human induced pluripotent stem cell (a human iPS cell) into a myocardial cell has been improved, and issues such as the purity and cost of the myocardial cell have been solved. However, it is known that the myocardial cell to be obtained is an immature myocardial cell in the human fetal period. As a method of maturing an immature myocardial cell, a stimulation method using a device or a physical stimulation method (for example, electrical stimulation or mechanical stimulation) has been reported (Non-Patent Documents 1 to 6). In these reports, after constructing a striped or ring-shaped myocardial tissue having a three-dimensional structure, a mechanical or electrical stimulation is applied from the outside to mature the myocardial tissue. However, the maturation method in which a mechanical stimulation or an electrical stimulation is applied from the outside has a disadvantage that cells are damaged and the myocardial tissue cannot be maintained for a long period of time and a control device is complicated and difficult to operate.

As a simpler method, a method of constructing a three-dimensional cell ring and promoting the maturation of myocardial cells is known (Patent Document 1). However, it is known that the myocardial cells mature by this method have very high metabolism and the provision of nutrients to cells in the middle of the tissue is insufficient in a case where the three-dimensional structure remains, and thus it is difficult to maintain the cells for a long period of time (Non-Patent Document 7).

### Prior Art Documents

### Non-Patent Documents

Non-Patent Document 1: Nature volume 556, pages 239-243 (2018)
Non-Patent Document 2: Nature Methods volume 10, pages 781-787 (2013)
Non-Patent Document 3: Nature Communications volume 11, Article number: 75 (2020)
Non-Patent Document 4: Scientific Reports volume 10, Article number: 6919 (2020)
Non-Patent Document 5: Circulation. 2017 May 9; 135 (19): 1832-1847
Non-Patent Document 6: Scientific Reports volume 4, Article number: 4781 (2014)
Non-Patent Document 7: Communications Biology volume 3, Article number: 122 (2020)

### Patent Documents

Patent Document 1: WO2018/124210

## SUMMARY OF THE INVENTION

An object to be achieved by an aspect of the present invention is to provide a production method of a myocardial cell layer and a myocardial cell layer, which allow myocardial cells to be matured without using a complicated control device and can be maintained for a long period of time. In addition, an object to be achieved by an aspect of the present invention is to provide a kit for evaluating a pharmaceutical candidate substance, a transplantation material, and an evaluation method of a pharmaceutical candidate substance, using the myocardial cell layer described above.

As a result of intensive studies to achieve the above object, the inventors of the present invention have found that the task of the provision of nutrients in addition to the maturation of myocardial cells can be achieved by culturing cells as a two-dimensional cell layer (single layer) instead of the three-dimensional cell ring as disclosed in WO2018/124210. In addition, although it was found that the myocardial cells are required to be cultured in a closed loop shape (for example, ring shape) for the maturation of the myocardial cells, the inventors of the present invention found that cells can be maintained for a long period of time in a case where a closed loop-shaped circumference and a conduction velocity of the myocardial cells (parameter related to contraction of the heart) satisfy a predetermined relationship. The present invention has been completed based on the above findings.

That is, according to an aspect of the present invention, the following inventions are provided.

<1> A production method of a myocardial cell layer, comprising the following steps (1) to (3),
(1) seeding a myocardial cell induced from a pluripotent stem cell in a single layer on a seeding surface having a shape shown in the following (1-1) to form a myocardial cell layer,
(1-1) in a case where spread of the seeding surface in a horizontal direction is a plane figure, a shape thereof is a shape obtained by hollowing out at least a part of a first plane figure of an optional shape having uniform spread in a second plane figure of another optional shape having uniform spread without contacting an outer periphery of the first plane figure of the optional shape, where an average value of a length of the outer periphery of the first plane figure and a length of an outer periphery of the second plane figure satisfies the following formula;

$$x \geq v/10$$

in the formula, x represents the average value of the length of the outer periphery of the first plane figure and the length of the outer periphery of the second plane figure, where a unit thereof is cm, and v represents a conduction velocity of the myocardial cell induced from the pluripotent stem cell, where a unit thereof is $cm \cdot s^{-1}$,
(2) circulating and proceeding at least one electrical signal or a calcium signal in the myocardial cell layer,
(3) culturing the cells for 8 days or more while maintaining a state of the (2).
<2> The production method according to <1>, in which the pluripotent stem cell is an induced pluripotent stem cell or an embryonic pluripotent stem cell.
<3> The production method according to <1> or <2>, in which an expression level of $\beta$-MHC in the myocardial cell layer is 10% or more of an expression level of a normal adult myocardial cell.
<4> The production method according to any one of <1> to <3>, in which an expression level of Connexin-43 in the myocardial cell layer is 1.5 times or more an expression level of control cells which are not treated with a circulation wave.
<5> The production method according to any one of <1> to <4>, in which a conduction velocity in the myocardial cell layer is 10 $cm \cdot s^{-1}$ or more.
<6> The production method according to any one of <1> to <5>, in which a VEGF secretion amount from the myocardial cell layer is 2 times or more a VEGF secretion amount from control cells which are not treated with a circulation wave.
<7> The production method according to any one of <1> to <6>, in which hypoxia resistance of the myocardial cell layer is improved as compared with control cells which are not treated with a circulation wave.
<8> The production method according to any one of <1> to <7>, in which the first plane figure is a circle, and the second plane figure is a circle having a diameter smaller than a diameter of the circle of the first plane figure.
<9> The production method according to any one of <1> to <7>, in which the first plane figure is a shape obtained by cutting a substantial rectangle from a circle to be in contact with an outer periphery of the circle, and the second plane figure is a shape of a Landolt ring, and
at least the part of the first plane figure is hollowed out in the second plane figure such that the rectangle is inserted between cuts of the Landolt ring.
<10> The production method according to any one of <1> to <7>, in which the first plane figure is a recessed type, and the second plane figure is a recessed type smaller than the first plane figure.
<11> The production method according to any one of <1> to <10>, in which an expression level of one or more myocardial maturation markers selected from TNNI3, ITGA7, IGF1, SCN5A, and KCNJ11 in the myocardial cell layer is 1.3 times or more an expression level in control cells which are not treated with a circulation wave.
<12> The production method according to any one of <1> to <11>, further comprising a step of performing co-culture with a fibroblast.
<13> The production method according to any one of <1> to <12>, in which the myocardial cell layer is formed on a biodegradable or non-biodegradable scaffold.
<14> The production method according to any one of <1> to <13>, in which a seeding density of the myocardial cells in the step (1) is $2 \times 10^5$ to $1 \times 10^6$ cells/cm$^2$.
<15> The production method according to any one of <1> to <14>, in which the pluripotent stem cell is a pluripotent stem cell having a gene mutation associated with a heart disease.
<16> A myocardial cell layer obtained by the production method according to any one of <1> to <15>.
<17> A myocardial cell layer having the following characteristics (1) to (5),

(1) the myocardial cell layer includes a myocardial cell induced from a pluripotent stem cell,

(2) the myocardial cell layer is a single layer,

(3) the myocardial cell layer satisfies at least one of the following (3-1) to (3-8),

(3-1) an expression level of $\beta$-MHC in the myocardial cell layer is 10% or more of a normal adult myocardial cell;

(3-2) a VEGF secretion amount from the myocardial cell layer is 2 times or more that of control cells which are not treated with a circulation wave;

(3-3) an expression level of Connexin-43 in the myocardial cell layer is 1.5 times or more that of the control cells which are not treated with the circulation wave;

(3-4) an expression level of TNNI3 in the myocardial cell layer is 2 times or more that of the control cells which are not treated with the circulation wave;

(3-5) an expression level of ITGA7 in the myocardial cell layer is 1.2 times or more that of the control cells which are not treated with the circulation wave;

(3-6) an expression level of IGF1 in the myocardial cell layer is 1.5 times or more that of the control cells which are not treated with the circulation wave;

(3-7) an expression level of SCN5A in the myocardial cell layer is 1.2 times or more that of control cells which are not treated with the circulation wave;

(3-8) an expression level of KCNJ11 in the myocardial cell layer is 1.3 times or more that of control cells which are not treated with the circulation wave;

(4) in a case where spread of a seeding surface in a horizontal direction is a plane figure, the shape thereof is a shape obtained by hollowing out at least a part of a first plane figure of an optional shape having uniform spread in a second plane figure of another optional shape having uniform spread without contacting an outer periphery of the first plane figure of the optional shape,

(5) an average value of a length of the outer periphery of the first plane figure and a length of an outer periphery of the second plane figure satisfies the following formula,

$$x \geq v/10$$

in the formula, x represents the average value of the length of the outer periphery of the first plane figure and the length of the outer periphery of the second plane figure, where a unit thereof is cm, and v represents a conduction velocity of the myocardial cell induced from the pluripotent stem cell, where a unit thereof is $cm \cdot s^{-1}$.

<18> A kit for evaluating a pharmaceutical candidate substance, comprising the myocardial cell layer according to <16> or <17>.

<19> A transplantation material comprising the myocardial cell layer according to <16> or <17>.

<20> An evaluation method of a pharmaceutical candidate substance, comprising:

(1) bringing the myocardial cell layer according to <16> or <17> into contact with a candidate substance;

(2) detecting an electrical signal or a calcium signal of the myocardial cell layer in contact with the candidate substance; and

(3) comparing the electrical signal or the calcium signal, which is detected in the step (2), with a control electrical signal or a control calcium signal.

According to an aspect of the present invention, it is possible to provide a myocardial cell layer, in which myocardial cells can be matured and maintained for a long period of time without using a complicated control device. The myocardial cell layer according to an aspect of the present invention can be used as a transplantation material or for evaluation of a pharmaceutical candidate substance.

**BRIEF DESCRIPTION OF THE DRAWINGS**

Fig. 1 shows a shape of a PDMS sheet.

Fig. 2 shows a shape of a culture container.

Fig. 3 shows a relationship between a beating frequency and a stability of a circulation wave in a case where a myocardial cell B is seeded in a culture container.

Fig. 4 shows a relationship between a beating frequency and a stability of a circulation wave in a case where a myocardial cell C having various purities is seeded in a culture container.

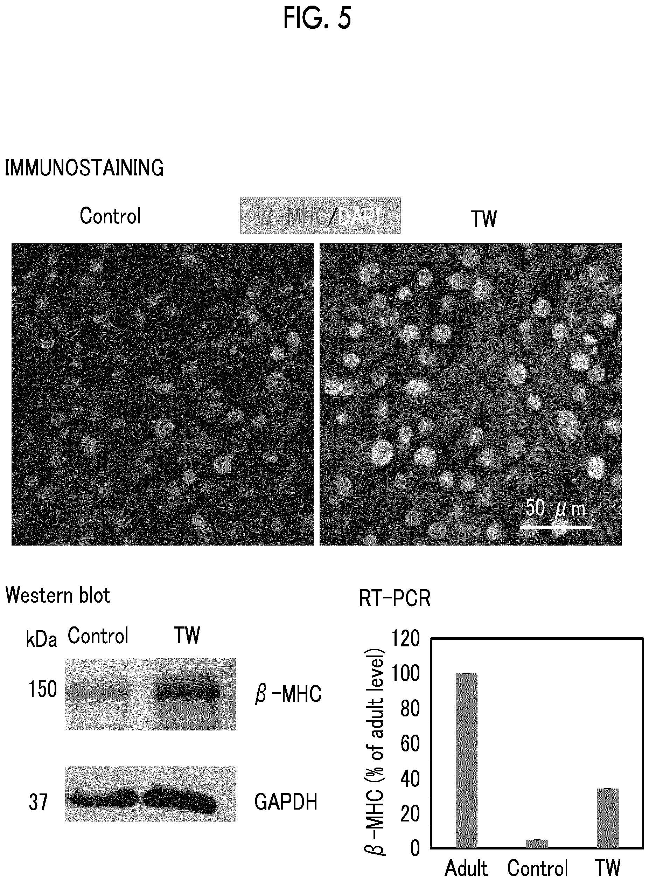

Fig. 5 shows results of measuring expression of $\beta$-MHC in the myocardial cell A.

Fig. 6 shows results of measuring expression of Connexin-43 in the myocardial cell A.

Fig. 7 shows results of measuring a conduction velocity of the myocardial cell A.

Fig. 8 shows results of measuring a VEGF secretion amount of the myocardial cell A.

Fig. 9 shows results of measuring hypoxia resistance of the myocardial cell A.

Fig. 10 shows results of confirming a circulation wave and a beating frequency in a case where the myocardial cell C is cultured on a scaffold.

Fig. 11 shows results of confirming expression of a myocardial maturation marker in a case where the myocardial cell C is cultured on a scaffold.

Fig. 12 shows results of measuring an electrical signal in a case where the myocardial cells B are cultured on different scaffolds.

Fig. 13 shows results of measuring an electrical signal, a QT interval, and a beating frequency in a case where the myocardial cell C is cultured on a scaffold.

Fig. 14 shows results of evaluating a drug E-4031 using the myocardial cell C.

Fig. 15 shows results of evaluating a drug Ranolazine using the myocardial cell C.

Fig. 16 shows results of evaluating a drug Mexiletine using the myocardial cell C.

Fig. 17 shows results of evaluating a drug Isoproterenol using the myocardial cell C.

Fig. 18 shows results of evaluating a drug Aspirin using the myocardial cell C.

Fig. 19 shows results of evaluating a drug Verapamil using the myocardial cell C.

## DESCRIPTION OF THE PREFERRED EMBODIMENTS

Hereinafter, the contents of embodiment of the present invention will be described in detail. In the present specification, a numerical value range indicated by using "to" means a range including numerical values described before and after the "to" as a minimum value and a maximum value, respectively.

<Production method of myocardial cell layer>

The production method of a myocardial cell layer according to the embodiment of the present invention includes the following steps (1) to (3).

(1) seeding a myocardial cell induced from a pluripotent stem cell in a single layer on a seeding surface having a shape shown in the following (1-1) to form a myocardial cell layer,

(1-1) in a case where spread of the seeding surface in a horizontal direction is a plane figure, a shape thereof is a shape obtained by hollowing out at least a part of a first plane figure of an optional shape having uniform spread in a second plane figure of another optional shape having uniform spread without contacting an outer periphery of the first plane figure of the optional shape, where an average value of a length of the outer periphery of the first plane figure and a length of an outer periphery of the second plane figure satisfies the following formula;

$$x \geq v/10$$

in the formula, x represents the average value of the length of the outer periphery of the first plane figure and the length of the outer periphery of the second plane figure, where a unit thereof is cm, and v represents a conduction velocity of the myocardial cell induced from the pluripotent stem cell, where a unit thereof is $cm \cdot s^{-1}$;

(2) circulating and proceeding at least one electrical signal or a calcium signal in the myocardial cell layer,

(3) culturing the cells for 8 days or more while maintaining a state of the (2).

The pluripotent stem cell means a cell having pluripotency, which enables differentiation into all cells that constitute an adult, and a self-replication ability, which can maintain the pluripotency even after cell division. Examples of the pluripotent stem cell include an induced pluripotent stem cell (iPS cell), an embryonic stem cell (ES cell), or an embryonic germ cell (EG cell). The pluripotent stem cell is preferably an induced pluripotent stem cell (iPS cell) or an embryonic stem cell (ES cell).

The species of the pluripotent stem cell is not particularly limited, but is preferably a mammal, more preferably a rodent or a primate, and further more preferably a primate. A monkey or human pluripotent stem cell, particularly a monkey or human ES cell, and an iPS cell can be suitably used. In some embodiments, the pluripotent stem cell is a human iPS cell.

A method of inducing a myocardial cell from a pluripotent stem cell is known and examples thereof include methods disclosed in WO2013/111875A, WO2015/182765A, WO2020/027278A, and Minami I, et al., Cell Rep. 2012 Nov 29; 2(5): 1448-60. In addition, the myocardial cells induced from pluripotent stem cells may be commercially available cells (for example, iCell (registered trademark) Cardiomyocytes (manufactured by FUJIFILM Cellular Dynamics, Inc.), iCell (registered trademark) Cardiomyocytes[2] (manufactured by FUJIFILM Cellular Dynamics, Inc.) as myocardial cells derived from ES cells or iPS cells.

In addition, as the pluripotent stem cell, a pluripotent stem cell having a gene mutation associated with a heart disease may be used. By inducing myocardial cells using a pluripotent stem cell having a mutation of a gene associated with a heart disease (for example, MYBPC3 gene, DSG2 gene, TNNI3 gene, or the like), a heart disease model can be established. Such a heart disease model can be used for drug discovery research.

As an example, it is possible to use a myocardial cell induced from a pluripotent stem cell by the method disclosed in WO2015/182765A. In brief, by a method including (a) step of culturing a pluripotent stem cell in a culture medium containing a WNT signal activator and a PKC activator; and (b) step of culturing the cells obtained in the step (a) in a culture medium including a WNT signal inhibitor, an Src inhibitor, and an EGF receptor inhibitor, it is possible to differentiate and induce myocardial cells from pluripotent stem cells.

The WNT signal activator means a substance that activates a WNT signal pathway. Examples of the WNT signal activator include a GSK3β inhibitor such as BIO and CHIR99021, and TWS119. The WNT signal inhibitor means a substance that inhibits the WNT signal pathway. Examples of the WNT signal inhibitor include a compound of Formula (I) or a salt thereof, and compounds such as IWP2, IWP4, XAV939, and IWR1, which are disclosed in the pamphlet of WO2012/026491A.

The PKC activator means a substance that activates protein kinase C (PKC) or a signal transduction pathway downstream thereof. Examples of the PKC activator include Phorbol 12-myristate 13-acetate (PMA), Prostratin, Bryostatin 1, Bryostatin 2, FR236924, (-)-Indolactam V, PEP005, Phorbol 12,13-dibutyrate, SC-9, SC-10, 1-oleoyl-2-acetyl-sn-glycerol, 1-O-hexadecyl-2-O-arachidonyl-sn-glycerol, 1,2-Dioctanoyl-sn-glycerol, PIP2, Resiniferatoxin, Phorbol 12,13-dihexanoate, Mezerein, Ingenol 3-Angelate, RHC-80267, DCP-LA, Lipoxin A4, and the like.

The Src inhibitor means a substance that inhibits Src, which is a tyrosine kinase, or a signal transduction pathway downstream thereof. Examples of the Src inhibitor include A419259, SU6656, PP1, 1-naphthyl PP1, PP2, indirubin-3'-(2,3-dihydroxypropyl)-oximether, TX-1123, Src kinase inhibitor I (CAS 179248-59-0), AZM475271, Bosutinib, Herbimycin A, KB SRC 4, MNS, PD166285, TC- S7003, and the like.

An EGF receptor inhibitor (also referred to as an EGFR inhibitor) means a substance that inhibits signal transduction from the EGF receptor. Examples of the EGF receptor inhibitor include AG1478, Gefitinib, Afatinib, ARRY334543, AST1306, AZD8931, BIBU1361, BIBX1382, BPDQ, BPIQ-I, BPIQ-II, Canertinib, CL-387, 785, CUDC101, Dacomitinib, Vandetanib, EGFR Inhibitor III (N-(4-((3,4-dichloro-6-fluorophenyl)amino)-quinazolin-6-yl)-2-chloroacetamide, CAS 733009-42-2), EGFR/ErbB-2 Inhibitor (4-(4-benzyloxyanilino)-6,7-dimethoxyquinazoline, CAS 179248-61-4), Erlotinib, GW583340, GW2974, HDS029, Lapatinib, WHI-P154, OSI-420, PD153035, PD168393, PD174265, Pelitinib, Compound 56, XL657, PP3, AG-490, AG555, Tyrphostin B42, Tyrphostin B44, AG556, AG494, AG825, RG-13022, DAPH, EGFR Inhibitor (cyclopropane carboxylic acid-(3-(6-(3-trifluoromethyl-phenylamino)-pyrimidin-4-ylamino)-phenyl)-amide, CAS 879127-07-8), Erbstatin Analog (methyl 2,5-dihydroxycinnamate, CAS 63177-57-1), JNJ28871063, Tyrphostin 47, Lavendustin A, Lavendustin C, Lavendustin C methyl ester, LFM-A12, TAK165, TAK285, Tyrphostin 51, Tyrphostin AG183, Tyrphostin AG528, Tyrphostin AG99, Tyrphostin RG14620, WZ3146, WZ4002, WZ8040, buteine, Tyrphostin AG112, and the like. In some aspects, the EGF receptor inhibitor is an EGF receptor inhibitor having a quinazoline-based skeleton, for example, AG1478, Gefitinib, Afatinib, ARRY334543, AST1306, AZD8931, BIBU1361, BIBX1382, BPDQ, BPIQ-I, BPIQ-II, Canertinib, CL-387,785, CUDC101, Dacomitinib, Vandetanib, EGFR Inhibitor III (CAS 733009-42-2), EGFR/ErbB-2 Inhibitor (CAS 179248-61-4), Erlotinib, GW583340, GW2974, HDS029, Lapatinib, WHI-P154, OSI-420, PD153035, PD168393, PD174265, Pelitinib, Compound 56, or XL657. The EGF receptor inhibitor is available from Santa Cruz Biotech and the like.

In an example, the culture medium of the step (b) contains two or more of WNT signal inhibitors, where one of the two or more of WNT signal inhibitors is any one of the compounds of Formula (I) or a salt thereof, and other WNT signal inhibitors are one or more compounds selected from IWP2, XAV939, and IWR1, particularly XAV939.

In an example, the WNT signal activator is BIO or CHIR99021, particularly CHIR99021.

In an example, the PKC activator is PMA or prostratin, particularly PMA.

In an example, the Src inhibitor is A419259 or SU6656, particularly A419259.

In an example, the EGF receptor inhibitor is AG1478 or Gefitinib, particularly AG1478.

In an example, in the steps (a) and (b), the cells are cultured by suspension culture. In an example, the step (a) is carried out for 1 to 3 days, and subsequently the step (b) is carried out for 2 to 13 days, preferably for 3 to 10 days, more preferably for 4 to 10 days, and further more preferably for 4 to 8 days, immediately after the end of the step (a) or 1 to 2 days after the end of the step (a).

As another example of inducing the myocardial cell from the pluripotent stem cell, it is possible to use the myocardial cell induced from the pluripotent stem cell by the method disclosed in WO2020/027278. In brief, by a method including (c) step of forming a plurality of embryoid bodies by performing spheroid culture of a pluripotent stem cell, and;

(d) step of differentiating and inducing the plurality of embryoid bodies obtained in the step (c) into myocardial cells by providing thereof for a three-dimensional suspension culture, it is possible to differentiate and induce myocardial cells.

The spheroid culture of the step (c) can be carried out generally for 3 to 5 days, and preferably 4 days. The three-dimensional suspension culture of the step (d) can be carried out generally for 9 to 15 days, preferably 10 to 14 days, more preferably 11 to 13 days, and further more preferably 12 days.

In the step (c), a culture medium containing BMP-4, activin A, and bFGF can be used. More specifically, a method of carrying out culturing under the following conditions can be mentioned, for example.

Day 0 to: BMP-4 final concentration of 2 ng/ml
Day 1 to: Activin A final concentration of 12 ng/ml, BMP-4 final concentration of 10 ng/ml, bFGF final concentration of 10 ng/ml

In the step (d), a culture medium containing VEGF, IWP-3, SB431542, Dorsomorphin, and bFGF can be used. More specifically, a method of carrying out culturing under the following conditions can be mentioned, for example.

Day 4 to: VEGF final concentration of 10 ng/ml, IWP-3 final concentration of 1 $\mu$M, SB431542 final concentration of 5.4 $\mu$M, Dorsomorphin final concentration of 3 $\mu$M
Day 8 to: VEGF final concentration of 10 ng/ml, bFGF final concentration of 5 ng/ml

At least a part of the myocardial cells induced from the pluripotent stem cells may express a gene of calcium sensor protein. The calcium sensor protein is not particularly limited as long as it is a protein that emits a signal such as luminescence in response to a change in an intracellular calcium concentration, and luminescent proteins such as Aequorin and Aequorin-GFP fusion protein or fluorescent proteins such as Cameleon, FIP-CBSM, TN-L15, YC6.1, F2C, Camgaroo, G-CaMP, and Pericam are included. In some embodiments, the calcium sensor protein is G-CaMP, which a calcium sensor protein to which a green fluorescent protein (EGFP), carmodulin (CaM), and a myosin light chain fragment (M13) are bound by genetic engineering, or a variant thereof, for example, G-CaMP, GCaMP1.6, GCaMP2, GCaMP3, GCaMP6f, GCaMP6m, GCaMP6s, and particularly GCaMP3.

In the embodiment of the present invention, myocardial cells induced from pluripotent stem cells are seeded in a single layer on a seeding surface having a shape shown in the following (1-1) to form a myocardial cell layer.

(1-1) in a case where spread of the seeding surface in a horizontal direction is a plane figure, a shape thereof is a shape obtained by hollowing out at least a part of a first plane figure of an optional shape having uniform spread in a second plane figure of another optional shape having uniform spread without contacting an outer periphery of the first plane figure of the optional shape, where an average value of a length of the outer periphery of the first plane figure and a length of an outer periphery of the second plane figure satisfies the following formula;

$$x \geq v/10$$

In the formula, x represents an average value of a length of the outer periphery of the first plane figure and a length of the outer periphery of the second plane figure, where a unit thereof is cm, v represents a conduction velocity of the myocardial cells induced from the pluripotent stem cell, and a unit thereof is cm·s$^{-1}$.

In the embodiment of the present invention, the task of the provision of nutrients can be achieved by culturing myocardial cells induced from pluripotent stem cells in a single layer (two-dimensional). In addition, there is an advantage that the myocardial cells induced from the pluripotent stem cells can be easily collected and stored, or an advantage that the myocardial cells induced from the pluripotent stem cells can be easily fused into a three-dimensional (3D) scaffold material.

In a case where the myocardial cell induced from the pluripotent stem cell is cultured in a single layer (two-dimensional), the myocardial cell may be co-cultured together with a cell other than the myocardial cell, for example, a fibroblast. It is preferable to co-culture the myocardial cell together with a cell other than the myocardial cell, for example, a fibroblast, rather than to culture only the myocardial cell. A proportion of the myocardial cell (purity of myocardial cell) with respect to all the cells in a case of carrying out co-culture is preferably 50% to 90%, more preferably 60% to 90%, and further more preferably 70% to 90%.

For the maturation of the myocardial cell induced from the pluripotent stem cell, it is required that the myocardial cell layer has a shape of a closed loop (as an example, a ring, but not limited to the ring). Specifically, the shape of the closed loop means that a shape in a case where spread of the myocardial cell layer in the horizontal direction is defined as a plane figure is a shape obtained by hollowing out at least a part of a first plane figure of an optional shape having uniform spread in a second plane figure of another optional shape having uniform spread without contacting an outer periphery of the first plane figure of the optional shape. By culturing the myocardial cell induced from the pluripotent stem cell in a state of the closed loop shape as described above, a phenomenon of high-speed propagation of an active potential is spontaneously induced (= induction of spontaneous circulation wave, (traveling wave, TW)), and by continuing the culture in such a state, the myocardial cell becomes mature.

There is a case where the myocardial cell induced from the pluripotent stem cell differs in characteristics or purity due to the difference in a production method or differentiation and induction method of the pluripotent stem cell to be used. One of the important characteristics regarding the formation and maturation of the myocardial cell induced from the pluripotent stem cell is a conduction velocity of the myocardial cell (a parameter related to the beat rate of the myocardium). In the embodiment of the present invention, it was found that a cell having a large conduction velocity of the myocardial cell is required to have a circumference of the closed loop.

More specifically, in a case where spread of the seeding surface in the horizontal direction is a plane figure, it is required that a shape thereof is a shape obtained by hollowing out at least a part of a first plane figure of an optional shape having uniform spread in a second plane figure of another optional shape having uniform spread without contacting an outer periphery of the first plane figure of the optional shape, and an average value of a length of the outer periphery of the first plane figure and a length of the outer periphery of the second plane figure satisfies the following formula.

$$x \geq v/10$$

In the formula, x represents an average value of a length of the outer periphery of the first plane figure and a length of the outer periphery of the second plane figure, where a unit thereof is cm, v represents a conduction velocity of the myocardial cells induced from the pluripotent stem cell, and a unit thereof is $cm \cdot s^{-1}$.

It is preferable that the formula $x \geq v/9$, $x \geq v/8$, $x \geq v/7$, or $x \geq v/6$ is satisfied, and it is more preferable that the formula $x \geq v/6$ is satisfied.

The shape of the closed loop is not particularly limited as long as the average value of the length of the outer periphery of the first plane figure and the length of the outer periphery of the second plane figure satisfies the above formula.

The closed loop shape of the myocardial cell layer is a shape obtained by hollowing out at least a part of a first plane figure in a second plane figure of another optional shape having uniform spread without contacting an outer periphery of the first plane figure of the optional shape.

A first example of the closed loop shape is a case where the first plane figure is a circle, and the second plane figure is a circle having a diameter smaller than a diameter of a circle of the first plane figure.

As a second example of the closed loop shape, there is a case where the first plane figure is a shape obtained by cutting a substantial rectangle from a circle to be in contact with the outer periphery of the circle, the second plane figure is a shape of a Landolt ring, and at least a part of the first plane figure is hollowed out from the second plane figure such that the rectangle is inserted between cuts of the Landolt ring.

As a third example of the closed loop shape, there is a case where the first plane figure is a recessed type, and the second plane figure is a recessed type smaller than the first plane figure.

As other examples of the closed loop shape, there is a case where the first plane figure is (1) a plane figure formed of curves (for example, circle and ellipse), (2) a plane figure formed of straight lines (for example, triangle, quadrangle, pentagon, hexagon), (3) a plane figure formed of curves and straight lines (for example, semicircle and Reuleaux polygon), or (4) a figure obtained by superimposing the above (1) to (3), and the second plane figure is any one of (1) to (4) smaller than the first plane figure.

A container for culturing the myocardial cells induced from the pluripotent stem cells is a container having at least one convex portion in an inner bottom portion. The inner bottom portion of the culture container means a part that forms an inner bottom surface of the culture container, and a part that forms a surface on a side on which the myocardial cells are received and cultured. The convex portion present in the inner bottom portion of the culture container is formed so that the myocardial cell layer can have a closed loop shape described above.

The convex portion present in the inner bottom portion of the culture container may be integrated with a main body part or may be separate from the main body part. In a case of being separate, the convex portion may be adhered to the main body part or may be placed on the main body part without being adhered thereto. For the adhesion, a polymer exemplified as a material of the convex portion, or a commercially available biocompatible gluing agent, for example, a silicone adhesive can be used.

The main body part of the culture container may be a container generally used for cell culture and is not particularly limited thereto. A shape of the main body part may be a petri dish, a plate, a bottle, a chamber, a multi-well plate (for example, a 6-, 12-, 24-, 48-, 96-, or 384-well plate), or the like. A material of the main body part is not particularly limited. Specifically, examples thereof can include an inorganic material such as metal, glass, and silicone, and an organic material represented by a plastic (for example, polystyrene resin, polyethylene resin, polypropylene resin, acrylonitrile/butadiene/styrene resin (ABS resin)), nylon, an acrylic resin, fluororesin, polycarbonate resin, polyurethane resin, methyl pentene resin, phenol resin, melamine resin, epoxy resin, vinyl chloride resin, polytetrafluoroethylene tetrafluoroethylene resin). The main body part may be a cell culture container that is generally marketed as a cell culture container or can be produced by a method known to those skilled in the art. For example, in a case where the main body part made of a plastic material is produced, the main body part can be produced by a practical forming method, for example, injection molding. In some embodiments, the main body part is a plastic petri dish or a multi-well plate.

A material of the convex portion is not particularly limited as long as the material is a substance which does not adversely affect cell culture. For example, the material may be selected from the same materials as those of the main body part, or may be the same as or different from the material of the main body part. In addition, in addition to the above-described material of the main body part, the material of the convex portion is selected from a polymer such as polydimethylsiloxane (PDMS), polymethylmethacrylate (PMMA), polyethylene terephthalate (PET), polyamide (PA) and polymethylglutarimide (PMGI), polyvinyl alcohol (PVA), polyethylene glycol (PEG), polyethylene vinyl acetate (PEVA), and polyethylene oxide (PEO), but is not limited thereto. As an example, a material of the convex portion is PDMS.

An inner surface of the main body part may have cell adhesiveness or non-cell adhesiveness. At least an inner surface part of the main body part may be formed of a substance having cell adhesiveness or non-cell adhesiveness. The material of the main body part may be a cell-adhesive or non-cell-adhesive substance, and at least an inner surface part of the main body part may be coated with a substance having cell adhesiveness or non-cell adhesiveness. In an example, the inner surface of the main body part has non-cell adhesiveness.

In addition, a surface of the convex portion may have cell adhesiveness or non-cell adhesiveness. At least the surface part of the convex portion may be formed of a substance having cell adhesiveness or non-cell adhesiveness. A material of the convex portion may be a substance having cell adhesiveness or non-cell adhesiveness, and at least a surface part of the convex portion may be coated with a substance having cell adhesiveness or non-cell adhesiveness. In some embodiments, the surface of the convex portion has non-cell adhesiveness.

Examples of the substance having non-cell adhesiveness for coating include celluloses such as 2-methacryloyloxyethyl phosphorylcholine (MPC) polymer, methyl cellulose, hydroxyethyl cellulose, hydroxypropyl cellulose, hydroxypropyl methyl cellulose, and carboxymethyl cellulose sodium; polyethylene oxide; carboxyvinyl polymer; polyvinylpyrrolidone; polyethylene glycol; polyamide such as polyacrylamide and poly N-isopropylacrylamide; polysaccharides such as chitin, chitosan, hyaluronic acid, alginic acid, starch, pectin, carrageenan, guar gum, gum Arabic, and dextran; albumin and derivatives thereof.

Examples of the substance having cell adhesiveness for coating include a positively charged polymer such as poly L-lysine and poly L-omithine, and an extracellular substrate such as Matrigel (trademark), hyaluronic acid, laminin, fibronectin, vitronectin, and collagen.

An outer frame may be disposed around the convex portion. In that case, myocardial cells are seeded and cultured between the convex portion and the outer frame. A material of the outer frame can be selected according to the main body part and the convex portion of the culture container, and may be the same as or different from that of the main body part or the convex portion. The outer frame may be integrated with the main body part or may be separate from the main body part. In the case of being separate, the outer frame may be adhered to the main body part. For the adhesion, the polymer exemplified as the material of the convex portion, or a commercially available biocompatible gluing agent, for example, a silicone adhesive can be used.

For an inner side of the outer frame, the outer frame may be present around the convex portion. A dimension of the inner side of the outer frame can be selected such that there is a sufficient area for forming a myocardial cell layer between the convex portion and the outer frame. For example, the dimension can be configured such that there is a distance of about 0.01 mm to about 10 mm, about 1 mm to about 9 mm, or about 4 mm to about 6 mm, for example, about 2.5 mm between the convex portion and an inner wall of the outer frame. The shape and the dimension of an outer side of the outer frame are not particularly limited as long as the shape and the dimension are within the main body part of the culture container. Several numbers of the outer frames may be present in one culture container. In some embodiments, one convex portion and one outer frame are present in one culture container. In some embodiments, a plurality of convex portions are present in one culture container, and each one outer frame is present around each convex portion.

In some embodiments, the culture container includes no outer frame. In that case, the shape and the dimension of a side wall portion of the culture container can be selected such that there is a sufficient area for forming a myocardial cell layer between the convex portion and the side wall portion. For example, the shape and the dimension can be configured such that there is a distance of about 0.01 mm to about 10 mm, about 1 mm to about 9 mm, or about 4 mm to about 6 mm, for example, about 2.5 mm between the convex portion and the side wall portion.

A height of the convex portion may be appropriately determined depending on the size of the main body part and the like, and is, for example, about 0.1 mm to about 10 mm, 0.1 mm to about 5 mm, about 1 mm to about 5 mm, or about 3 mm to about 5 mm, for example, about 2 to 4 mm. A height of the outer frame may be any one as long as the height is enough to fill the inside of the outer frame with a culture solution, and is appropriately determined depending on the size of the main body part and the like, but is not particularly limited thereto. The height of the outer frame may be the same as or different from the height of the convex portion.

The culture medium, the culture method, and the culture conditions that are used for producing the myocardial cell layer may be those that are generally used for culturing myocardial cells, and are not particularly limited thereto. Examples of the culture medium that is used in the above method include a culture medium including 40% DMEM, 40% IMDM, 20% fetal calf serum, a 1% MEM non-essential amino acid solution, 0.1% penicillin-streptomycin, and 0.5% L-glutamine.

The myocardial cell layer may be formed on a biodegradable or non-biodegradable scaffold.

By culturing myocardial cells using a culture container in which the surface (culture surface) is coated with a biodegradable or non-biodegradable scaffold, it is possible to produce a myocardial cell layer.

As the biodegradable scaffold, a biodegradable polymer can be used. Examples of a synthetic polymer include a polyester-based polymer, a polyamide-based polymer, and a polycyanoacrylate-based polymer, and polylactic acid, polyglycolic acid, or a glycolic acid-lactic acid copolymer is preferable. As the biodegradable polymer, a biological polymer can also be used. Examples of the biological polymer include polysaccharides, proteins, nucleic acids, glycoproteins, and the like. Specifically, an extracellular matrix such as collagen, laminin, fibronectin, elastin, tenascin, hyaluronic acid, fibrin, and proteoglycan can be used.

Examples of the non-biodegradable scaffold include polyethylene terephthalate, polypropylene, polystyrene, polycarbonate, nylon, Teflon (registered trademark), ABS, and the like.

Examples of the shape of the biodegradable or non-biodegradable scaffold include a film, a mesh, a woven fabric, a non-woven fabric, and the like.

The seeding density of the myocardial cells in the step (1) is not particularly limited as long as a myocardial cell layer can be formed, but is preferably $2 \times 10^5$ to $6 \times 10^6$ cells/cm$^2$, and more preferably $4 \times 10^5$ to $1 \times 10^6$ cells/cm$^2$.

Examples of the culture method include an adhesion culture method.

Exchange of the culture medium may be performed, for example, once in 1 to 7 days, and, for example, once in 4 days, 3 days, or 2 days. A culture period may be a period sufficient to form a myocardial cell layer, and is, for example, 0.5 days to 3 months, 1 day to 21 days, 2 days to 14 days, 5 days to 10 days, or 6 days to 8 days.

As described above, by culturing the myocardial cells in a state of a closed loop shape that satisfies a predetermined condition of $x \geq v/10$ described above, a spontaneous circulation wave (TW) is induced in the myocardial cell layer, as described above. In other words, at least one electrical signal or calcium signal can be circulated and proceeded (also referred to as a circulation wave) in the myocardial cell layer. In the embodiment of the present invention, while maintaining a state in which an electrical signal or a calcium signal circulates and proceeds, that is, while maintaining a state in which the TW proceeds, the myocardial cells are cultured for 8 days or more (for example, 8 days, 9 days, 10 days, 11 days, 12 days, 13 days, 14 days, or 14 days or more).

The electrical signal means a change in membrane potential of the myocardial cells. The calcium signal means $Ca^{2+}$ oscillation of the myocardial cells. The electrical signal and the calcium signal circulate in a cell layer by proceeding in a certain direction in the myocardial cell layer. In some embodiments, the electrical signal, the calcium signal, and the beating of the myocardium show the same patterns. There is also a case where the circulation wave is one (that is, one electrical signal or calcium signal circulates and proceeds in the myocardial cell layer), there is also a case where the circulation wave are two, three, or more, and there is also a case where the number thereof during the culture fluctuates (for example, decrease). Although not limited by theory, it is seen that, in a case where a circulation wave is generated, myocardial cells contained in the myocardial cell layer adopt an alignment structure similar to those acknowledged in the heart of a living body, and the degree of maturity of the myocardial cells tends to increase. In addition, it is seen that there is a tendency that in a case where the number of the circulation waves increases, the orientation property and the degree of maturity of the myocardial cells increase. Accordingly, it is possible to obtain myocardial cells having a high degree of maturity by a simple operation. In the embodiment of the present invention, a myocardial cell layer in which at least one circulation wave is seen is selected.

It is known in the field that electrical or mechanical stimulation is applied to increase the degree of maturity of myocardial cells, and thus such a step may be carried out in the above-described method as well, but it is not necessary to carry out such a step. In some embodiments, the electrical or mechanical stimulation is not applied to the myocardial cell layer at a frequency equivalent to the physiological heart rate. In some embodiments, the myocardial cell layer is not subjected to any electrical stimulation or mechanical stimulation.

In the embodiment of the present invention, the electrical signal of the myocardial cell layer may be detected. By detecting the electrical signal, it is possible to evaluate the function and the maturity of the myocardial cells in the myocardial cell layer.

The electrical signal can be detected, for example, by bringing the myocardial cell layer into contact with a multiple electrode array. The multiple electrode array means an electrode in which a large number of microelectrodes, for example, 1 to 384, for example, 64 or 96 microelectrodes are disposed on a substrate. As the multiple electrode array (MEA) system that can measure the extracellular potential of cultured cells, a multiple electrode array system of Multi Channel Systems MCS GmbH (Germany), an MED system of Alfa Med Scientific, Inc., and the like are commercially available. The multiple electrode array system is a system in which electrical signals from a plurality of cells can be simultaneously observed by a large number of microelectrodes disposed on a substrate, or a response of cells to a stimulation can be observed by electrically applying stimulation to cells or tissues. By using the multiple electrode array system, it is possible to evaluate the function of cells without imparting damage to the cells. Specifically, by the multiple electrode array system, it is possible to measure an active potential waveform, and to evaluate the function of the myocardial cells based on the number of channels capable of detecting an electrical signal, a heart rate, a potential amplitude, FPD, the presence or absence of arrhythmia, and the like.

The myocardial cell layer can be easily taken out from the culture container by tweezers and the like, and the electrical signal can be measured by bringing the myocardial cell layer into contact with the multiple electrode array. Alternatively, by culturing the myocardial cells in a culture container in which a multiple electrode array and convex portions are disposed so that at least a part of the myocardial cell layer is formed on the multiple electrode array, the electrical signal of the myocardial cell layer may be measured.

In the embodiment of the present invention, the calcium signal of the myocardial cell layer may be detected. The calcium signal can be detected as a change in an intracellular calcium concentration of the myocardial cells. By analyzing the calcium signal, it is possible to evaluate the function and the degree of maturity of the myocardial cells in the myocardial cell layer.

The $Ca^{2+}$ oscillation can be detected by a standard method using, for example, a calcium detection reagent such as a calcium-sensitive dye. For example, the myocardial cells are cultured in a culture medium containing an intracellular $Ca^{2+}$ ion detection dye, and a change in the intracellular calcium level is measured. Examples of the intracellular $Ca^{2+}$ ion detection dye include Fura-2, bis-Fura2, Indo-1, Quin-2, Quin-2AM, benzothiaza-1, benzothiaza-2, Indo-5F, Fura-FF, BTC, Mag-Fura-2, Mag-Fura-5, Mag-Indo-1, Fluo-3, Rhod-2, Rhod-3, Fura-4F, Fura-5F, Fura-6F, Fluo-4, Fluo-5F, Fluo-5N, Oregon Green 488 BAPTA, Calcium Green, Calcein, Fura-C18, Calcium Green-C18, Calcium Orange, Calcium Crimson, Calcium Green-5N, Magnesium Green, Oregon Green 488 BAPTA-1, Oregon Green 488 BAPTA-2, X-Rhod-1, Fura Red, Rhod-5F, Rhod-5N, X-Rhod-5N, Mag-Rhod-2, Mag-X-Rhod-1, Fluo-5N, Fluo-5F, Fluo-4FF, Mag-Fluo-4, Aequorin, a dextran conjugate, or a derivative of these dyes, but are not limited thereto. Alternatively, in a case where at least a part of the myocardial cell layer expresses a gene of the above-described calcium sensor protein, the $Ca^{2+}$ oscillation can be detected even in a case where the intracellular $Ca^{2+}$ ion detection dye is not used. Here, the myocardial cell layer can contain myocardial cells expressing a gene of a calcium sensor protein in the number or proportion at which the $Ca^{2+}$ oscillation can be detected.

The fluorescence intensity or the luminescence amount of the intracellular $Ca^{2+}$ ion detection dye or the calcium sensor protein can be measured using, for example, a CCD camera, a luminometer, or other optional suitable optical systems. Examples of a commercially available optical system that can be used include Qimaging (Exiblue) and the like. Software such as ImageJ and Matlab can be used for the analysis of the $Ca^{2+}$ oscillation.

As an example, the beating of the myocardial cell, that is, spontaneous contraction may be detected. The beating of the myocardial cell can be detected by a standard electrophysiological method (for example, patch clamping), an elastic modulus detection device (for example, MicroTester (CellScale)), or a combination of these methods and devices.

In the myocardial cells contained in the myocardial cell layer in which one circulation wave is seen, the expression level of the β-MHC, which is a myocardial cell-specific marker showing the degree of maturity of the myocardial cell, is preferably about 10% or more, about 15% or more, about 20% or more, about 25% or more, or about 30% or more of normal adult myocardial cells. In addition, an expression level of β-MHC is about 60% or less, about 70% or less, about 80% or less, about 90% or less, or about 100% or less of an adult normal myocardial cell.

In the embodiment of the present invention, the expression of a myocardial maturation marker in the myocardial cells may be detected. By analyzing expression of the marker, it is possible to evaluate the degree of maturity of the myocardial cell. In some embodiments, the above-described method further includes a step of selecting a myocardial cell layer containing myocardial cells having high expression of a myocardial maturation marker.

Examples of the myocardial maturation marker include TNNI3, Integrin alpha 7 (ITGA7), IGF1, SCN5A, and KCNJ11. The expression of the myocardial cell-specific marker can be detected by a biochemical method or immunochemical method (for example, enzyme-linked immunosorbent assay, an immunohistochemical assay, and the like) in the related art. Alternatively, the expression of a nucleic acid encoding a myocardial cell-specific marker can be evaluated. Expression of a nucleic acid encoding a myocardial cell-specific marker can be confirmed by a molecular biological method (reverse transcription polymerase chain reaction (RT-PCR), hybridization analysis, or the like) that could be generally used in the past for amplification, detection, and analysis of mRNA. The nucleic acid sequence encoding the myocardial cell-specific marker is known, and is available through a public database such as GenBank, and thus the marker specific sequence used as a primer or a probe is easily determined.

The expression level of the myocardial maturation marker in the myocardial cell layer is preferably 1.3 times or more, more preferably 1.7 times or more, and further more preferably 2 times or more the expression level of the control cells which are not treated with the circulation wave. Alternatively, the expression level of the myocardial maturation marker in the myocardial cell layer is such that a value of fragments per kilobase of exon per million mapped reads) (FPKM) is 100 or more, preferably 150 or more, for TNNI3, 35 or more, preferably 40 or more, for ITGA7, 0.2 or more, preferably 0.4 or more, for IGF1, 18 or more, preferably 20 or more, for SCN5A, and 2.5 or more, and preferably 2.8 or more, for KCNJ11.

In the embodiment of the present invention, the expression of Connexin-43 in the myocardial cells may be detected. In some embodiments, the above-described method further includes a step of selecting a myocardial cell layer containing myocardial cells having high expression of Connexin-43. The expression of Connexin-43 can be analyzed in the same manner as in a case of the expression of the myocardial cell-specific marker.

The expression level of Connexin-43 in the myocardial cell layer is preferably 1.5 times or more, more preferably 1.7 times or more, and further more preferably 2 times or more the expression level of the control cells which are not treated with the circulation wave. It is expected that a high expression level of Connexin-43 improves an excitation conduction velocity between the myocardial cells and the adjacent cells.

The conduction velocity in the myocardial cell layer (conduction velocity of myocardial cells after being treated by the spontaneous circulation wave) is preferably 10 cm·s$^{-1}$ or more, more preferably 12 cm·s$^{-1}$ or more, and further more preferably 14 cm·s$^{-1}$ or more. It is expected that the function as the myocardial cell is high due to the high conduction velocity.

The VEGF secretion amount from the myocardial cell layer is preferably 2 times or more, more preferably 2.5 times or more, and further more preferably 3 times or more the VEGF secretion amount from the control cells which are not treated with the circulation wave. The VEGF secretion amount from the myocardial cell layer can be measured according to a common method by collecting a culture supernatant and performing enzyme-linked immuno sorbent assay (ELISA). It is expected that by improving the VEGF secretion amount, angiogenesis is promoted and the engraftment rate of myocardial cells after transplantation is improved.

It is preferable that the hypoxia resistance of the myocardial cell layer is improved as compared with control cells which are not treated with the circulation wave. The hypoxia resistance of the myocardial cell layer can be evaluated by culturing the myocardial cells in a low oxygen environment and then measuring the viability of the cells. It is expected that the engraftment rate of the myocardial cells after transplantation is improved by improving the hypoxia resistance.

<Myocardial cell layer>

According to the embodiment of the present invention, there is provided a myocardial cell layer obtained by the above-described production method of a myocardial cell layer according to the embodiment of the present invention.

According to the embodiment of the present invention, it is possible to further provide a myocardial cell layer having the following characteristics (1) to (5).

(1) the myocardial cell layer includes a myocardial cell induced from a pluripotent stem cell,
(2) the myocardial cell layer is a single layer,
(3) the myocardial cell layer satisfies at least one of the following (3-1) to (3-8),

(3-1) an expression level of β-MHC in the myocardial cell layer is 10% or more of a normal adult myocardial cell;
(3-2) a VEGF secretion amount from the myocardial cell layer is 2 times or more that of control cells which are not treated with a circulation wave;
(3-3) an expression level of Connexin-43 in the myocardial cell layer is 1.5 times or more that of the control cells which are not treated with the circulation wave;
(3-4) an expression level of TNNI3 in the myocardial cell layer is 2 times or more that of the control cells which are not treated with the circulation wave;
(3-5) an expression level of ITGA7 in the myocardial cell layer is 1.2 times or more that of the control cells which are not treated with the circulation wave;
(3-6) an expression level of IGF1 in the myocardial cell layer is 1.5 times or more that of the control cells which are not treated with the circulation wave;
(3-7) an expression level of SCN5A in the myocardial cell layer is 1.2 times or more that of control cells which are not treated with the circulation wave;
(3-8) an expression level of KCNJ11 in the myocardial cell layer is 1.3 times or more that of control cells which are not treated with the circulation wave;

(4) in a case where spread of a seeding surface in a horizontal direction is a plane figure, the shape thereof is a shape obtained by hollowing out at least a part of a first plane figure of an optional shape having uniform spread in a second plane figure of another optional shape having uniform spread without contacting an outer periphery of the first plane figure of the optional shape,

(5) an average value of a length of the outer periphery of the first plane figure and a length of an outer periphery of the second plane figure satisfies the following formula.

$$x \geq v/10$$

In the formula, x represents an average value of a length of the outer periphery of the first plane figure and a length of the outer periphery of the second plane figure, where a unit thereof is cm, v represents a conduction velocity of the myocardial cells induced from the pluripotent stem cell, and a unit thereof is cm·s$^{-1}$.

The above (1), (2), (4), and (5) are as described in the description of the production method of a myocardial cell layer.

The expression level of β-MHC in the myocardial cell layer is 10% or more, preferably 20%, and more preferably 30% of that of the normal adult myocardial cells.

The VEGF secretion amount from the myocardial cell layer is 2 times or more, preferably 3 times, and more preferably 4 times that of the control cells which are not treated with the circulation wave.

The expression level of Connexin-43 in the myocardial cell layer is 1.5 times or more, preferably 2 times, and more preferably 2.5 times that of the control cells which are not treated with the circulation wave.

The expression level of TNNI3 in the myocardial cell layer is 2 times or more, preferably 5 times, and more preferably 10 times that of the control cells which are not treated with the circulation wave.

The expression level of ITGA7 in the myocardial cell layer is 1.2 times or more, preferably 1.3 times, and more preferably 1.5 times that of the control cells which are not treated with the circulation wave.

The expression level of IGF1 in the myocardial cell layer is 1.5 times or more, preferably 2 times, and more preferably 3 times that of the control cells which are not treated with the circulation wave.

The expression level of SCN5A in the myocardial cell layer is 1.2 times or more, preferably 1.3 times, and more preferably 1.5 times that of the control cells which are not treated with the circulation wave.

The expression level of KCNJ11 in the myocardial cell layer is 1.3 times or more, preferably 1.4 times, and more preferably 1.5 times that of the control cells which are not treated with the circulation wave.

The above-described myocardial cell layer according to the embodiment of the present invention can be used as a transplantation material to the heart in the form of a cell sheet and the like.

<Evaluation method of pharmaceutical candidate substance and kit>

The embodiment of the present invention is to provide an evaluation method of a pharmaceutical candidate substance including:

(1) bringing a myocardial cell layer according to the embodiment of the present invention into contact with a candidate substance,
(2) detecting an electrical signal or a calcium signal of the myocardial cell layer in contact with the candidate substance, and
(3) comparing the electrical signal or the calcium signal detected in the step (2) with a control electrical signal or a control calcium signal.

In the step (1), for example, the myocardial cell layer is brought into contact with a candidate substance by adding the candidate substance to a culture medium of the myocardial cell layer. The candidate substance includes a candidate substance of an effective component of a pharmaceutical product in all regions. In the step (2), the electrical signal or the calcium signal of the myocardial cell layer in contact with the candidate substance is detected by the detection method of an electrical signal or a calcium signal. The control in the step (3) may be a myocardial cell layer before being brought into contact with the candidate substance, or may be a myocardial cell layer that is not in contact with the candidate substance. Alternatively, the control may be a myocardial cell layer that is not brought into contact with a substance known to have no cardiotoxicity or a substance having acceptable cardiotoxicity. As a result of the step (3), in a case where the electrical signal or the calcium signal of the myocardial cell layer in contact with the candidate substance is significantly different from the control electrical signal or the control calcium signal, the pharmaceutical candidate substance can be determined to have cardiotoxicity. In addition, to support the efficacy of the assay system, a myocardial cell layer in contact with a substance that decreases or increases the myocardial beating frequency or a substance that extends or shortens the QT interval can be used as a positive control.

As an example, as a pharmaceutical candidate substance, a treatment agent for a heart disease can be evaluated.

The heart disease means arrhythmia or a disease of a circulation system accompanied by arrhythmia. The arrhythmia includes accompanying arrhythmia and gradual arrhythmia. In the step (1), for example, the myocardial cell layer is brought into contact with a candidate substance by adding the candidate substance to a culture medium of the myocardial cell layer. The candidate substance includes a substance expected to have a treatment effect for a heart disease, for example, a substance expected to control the myocardial beating frequency or the QT interval. Before the step (1), a treatment for causing arrhythmia may be performed by bringing the myocardial cell layer into contact with a substance that decreases or increases the myocardial beating frequency or a substance that extends or shortens the QT interval. In the step (2), the electrical signal or the calcium signal of the myocardial cell layer in contact with the candidate substance is detected by the detection method of an electrical signal or a calcium signal. The control in the step (3) may be a myocardial cell layer before being brought into contact with the candidate substance, or may be a myocardial cell layer that is not in contact with the candidate substance (negative control). Alternatively, the control may be a myocardial cell layer in contact with a substance that decreases or increases the myocardial beating frequency or a substance that extends or shortens the QT interval (positive control). Depending on the necessity, the control myocardial cell layer may be subjected to a treatment of causing the same arrhythmia as in the myocardial cell layer to be tested.

As a result of the step (3), in a case where the electrical signal or the calcium signal of the myocardial cell layer in contact with the candidate substance is significantly different from the negative control electrical signal or the negative control calcium signal, or in a case where the electrical signal or the calcium signal of the myocardial cell layer in contact with the candidate substance shows the same tendency as that of the positive control electrical signal or the positive control calcium signal, it is determined that the candidate substance can be used for treating a heart disease.

Examples of the substance that decreases a myocardial beating frequency, which is used in the evaluation method of a pharmaceutical candidate substance include an adrenergic stimulating agent such as isoproterenol. Examples of the substance that increases the myocardial beating frequency include a β blocker such as propranolol. Examples of a substance that extends the QT interval include a HERG channel inhibitor such as E-4031. As the pharmaceutical candidate substance, a calcium channel blocker such as ranolazine, a sodium channel blocker such as mexiletine, verapamil, or the like can also be used.

In the evaluation method of a pharmaceutical candidate substance, a myocardial cell layer expressing a calcium sensor protein may be used to detect a calcium signal. In this case, the above method can be carried out without using a special detection reagent or electrode. In another aspect, the electrical signal of the myocardial cell layer may be detected by a multiple electrode array.

The embodiment of the present invention provides a kit for carrying out the evaluation method of a pharmaceutical candidate substance, which includes the myocardial cell layer. The kit may further contain a culture medium, a control substance, a calcium detection reagent, a multiple electrode array, a manual for using the kit, and the like.

The embodiment of the present invention will be described more specifically with reference to the following examples, but the embodiment of the present invention is not limited to the examples.

Examples

<Used cells>

Myocardial cell A:
Myocardial cells (a beating myocardial colony) induced from pluripotent stem cells were used according to a method disclosed in Examples (particularly, paragraphs 0064 and 0065 and Table 2) of WO2015/182765. As the pluripotent stem cells, human iPS cells (cell strain name: 253G1) were used.
Myocardial cell B:
Myocardial cells induced from pluripotent stem cells were used according to a method disclosed in WO2020/027278 (in particular, Example 3). As the pluripotent stem cells, human iPS cells (cell strain name: 253G1) were used.
Myocardial cell C:
Commercially available iCell (registered trademark) Cardiomyocytes[2] (manufactured by FUJIFILM Cellular Dynamics, Inc.) was used.

<Properties of used cells>

The myocardial cell A, the myocardial cell B, and the myocardial cell C have difference in iPS strains or the myocardial differentiation induction method used at the time of establishing each thereof, and thus characters of the obtained myocardium, particularly the conduction velocity, are different from one another. Specifically, the conduction velocity of each myocardial cell measured by a multiple electrode system (USB-ME64-System, Multi Channel Systems, Germany) is as follows.

Conduction velocity of myocardial cell A: 8 cm/s;
Conduction velocity of myocardial cell B: 30 cm/s;
Conduction velocity of myocardial cell C: 31 cm/s

In addition, each myocardial cell has a different purity. The purity of each myocardial cell is as follows. The purity of the myocardial cell was obtained by measuring a proportion of cells expressing TnT2, which is a myocardial cell-specific marker, with a flow cytometer. For example, a purity of 90% of the myocardial cell means that 90% of the measured cells express TnT2.

Purity of myocardial cell of myocardial cell A: 90%
Purity of myocardial cell of myocardial cell B: 50% to 90%
Purity of myocardial cell of myocardial cell C: 100%

Example 1: Preparation of culture container

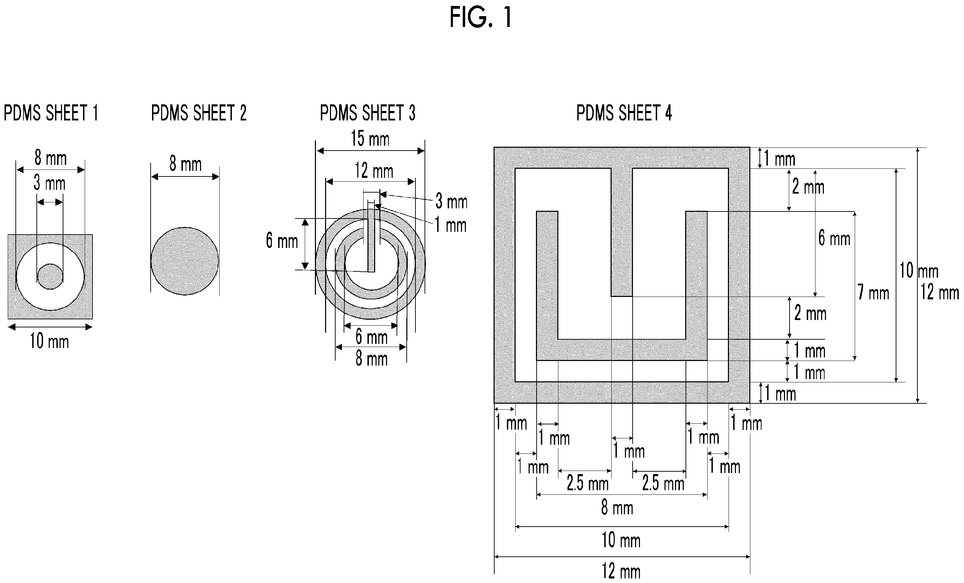

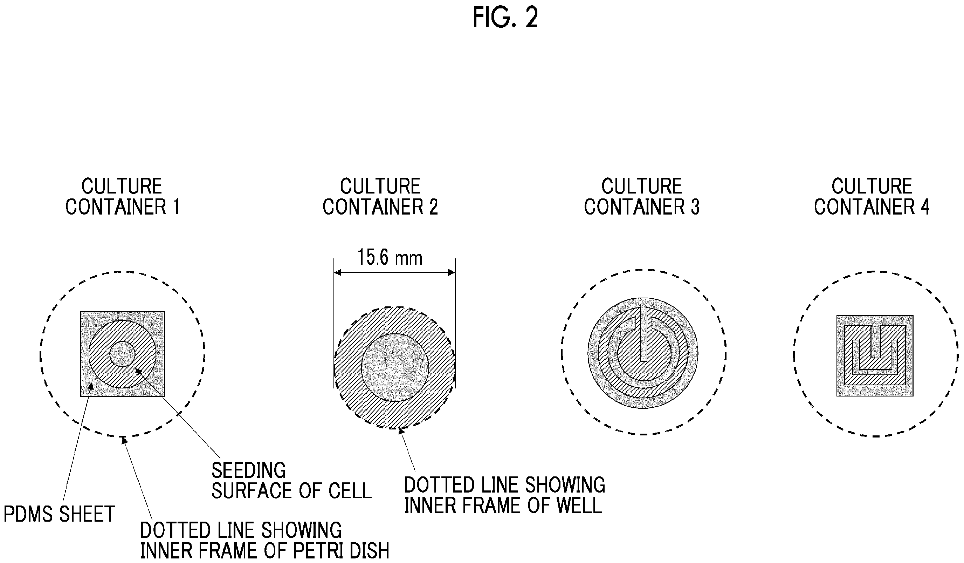

A PDMS (SYLGARD (registered trademark) 184, Dow Corning) sheet having a thickness of 4 mm was cut out into a shape shown in the PDMS sheets 1 to 4 in Fig. 1 using a tissue puncher (Fisher Scientific) and a cutter. Specifically, regarding the PDMS sheet 1, as shown in Fig. 1, a square PDMS sheet having a side of 10 mm with a circular hole having a diameter of 8 mm and a circular PDMS sheet having a diameter of 3 mm were created. Regarding the PDMS sheets 2 to 4, each of the PDMS sheets having the shapes shown in Fig. 1 was created. A silicone adhesive (one-pack condensation type RVT rubber (transparent) (Shin-Etsu Silicone)) was attached to one surface of the cut PDMS sheet, the PDMS sheets 1, 3, and 4 were adhered to a position shown in Fig. 2 of an adhesive culture petri dish (product name), and the PDMS sheet 2 was adhered to a position shown in Fig. 2 of a well (product name) of a 24-well plate. After being dried overnight, the resultant product was sterilized by UV irradiation for 30 minutes. As described above, culture containers 1 to 4 shown in Fig. 2 were prepared. In Fig. 2, a hatched portion shows a seeding surface of cells, and a dotted line shows an inner frame of a well of an adhesive culture petri dish or a 24-well plate.

Here, in a case where spread of the seeding surface of the culture container 1 in a horizontal direction is defined as a plane figure, the shape thereof is a shape obtained by hollowing out a circle having a diameter of 3 mm (second plane figure) from a circle (first plane figure) having a diameter of 8 mm without contacting an outer periphery of the first plane figure. In addition, a length of the outer periphery of the first plane figure is 25.12 mm, a length of the outer periphery of the second plane figure is 9.42 mm, and an average value of both ones is 17.27 mm. Regarding the culture devices 1 to 4, Table 1 shows the length of the outer periphery of the first plane figure, the length of the outer periphery of the second plane figure, and an average value of both ones.

[Table 1]

|  | Outer periphery of first plane figure (A) | Outer periphery of second plane figure (B) | Average value of A and B |
|---|---|---|---|
| Culture container 1 | 25.12 | 9.42 | 17.27 |
| Culture container 2 | 48.98 | 25.12 | 37.05 |
| Culture container 3 | 49.68 | 39.96 | 44.82 |
| Culture container 4 | 52 | 42 | 47 |
| (Unit: mm) | | | |

Example 2: Production of myocardial cell layer (use of myocardial cell B)

Myocardial spheres of the myocardial cells B induced from the pluripotent stem cells at 3 to 4 weeks were separated with a dissociation solution (0.05% Trypsin/EDTA). The separated myocardial cells B were dispersed at $1 \times 10^6$ cells/cm$^2$ in a 200 $\mu$l of myocardial culture solution including serum (culture medium including IMDM (Sigma-Aldrich, I3390), 1% MEM non-essential amino acid solution (Sigma-Aldrich, M7145), 1% penicillin/streptomycin (GIBCO, 15140), 2 mmol/L L-glutamine (Sigma-Aldrich, G7513), 0.001% 2-mercaptoethanol (GIBCO), and 0.005 mol/L NaOH), and scattered on the seeding surface of the cells in the culture containers 1 to 4 prepared in Example 1 to form a myocardial cell layer.

5 hours after the myocardial cells B were scattered on the seeding surface, 1 mL of the myocardial culture solution was added. After two days, the myocardial culture solution was exchanged to a myocardial culture solution containing no serum, and then the culture medium was exchanged at a frequency of once in four days.

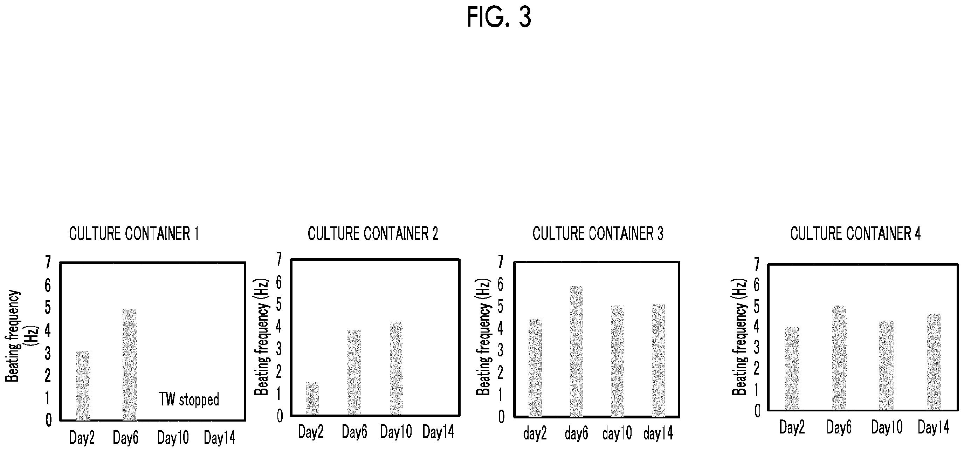

A day on which the myocardial cells B were scattered in the culture containers 1 to 4 created in Example 1 was defined as the 0th day (Day 0), and it was confirmed that the TW was generated in the myocardial cell layer under a microscope on the 2nd day (Day 2). A beating frequency was measured on the 2nd day (Day 2), the 6th day (Day 6), the 10th day (Day 10), and the 14th day (Day 14). Regarding the beating frequency, the beating of the myocardial cells was recorded with a CCD camera (DP74; Olympus), and the beating frequency was analyzed using Z-axis profile of ImageJ. to obtain data. The measurement results are shown in Fig. 3. In the culture container 1, in the myocardial cells B, the TW was stable up to Day 6 (beating frequency after Day 10 is not measured). In the culture container 2, the TW was stable up to Day 10 (beating frequency of Day 14 is not measured). In the culture containers 3 and 4, the TW was stable up to Day 14.

Example 3: Production of myocardial cell layer (use of myocardial cell A)

The myocardial colony of the myocardial cells A induced from the pluripotent stem cells at 3 to 4 weeks after the myocardial differentiation induction culture start was separated with a dissociation solution (0.1% collagenase type I, 0.25% trypsin, 1U/ml DNase I, 116 mmol/L NaCl, 20 mmol/L HEPES, 12.5 mmol/L NaH$_2$PO$_4$, 5.6 mmol/L glucose, 5.4 mmol/L KCl, and 0.8 mmol/L MgSO$_4$, pH 7.35). The obtained myocardial cells A were dispersed at $1 \times 10^6$ cells/cm$^2$ in 200 μl of a myocardial culture solution including serum (culture medium including IMDM (Sigma-Aldrich, I3390), 1% MEM non-essential amino acid solution (Sigma-Aldrich, M7145), 1% penicillin/streptomycin (GIBCO, 15140), 2 mmol/L L-glutamine (Sigma-Aldrich, G7513), 0.001% 2-mercaptoethanol (GIBCO), and 0.005 mol/L NaOH), and seeded on the seeding surface of the cells of the culture container 1 prepared in Example 1 to form a myocardial cell layer. The culture of the myocardial cell layer was carried out in the same manner as in Example 2. In the culture container 1, it was confirmed that the TW was stable up to Day 14.

A period during which the stability of the TW was maintained differed depending on the combination of the kind of myocardial cells used and the culture container. The results of Examples 2 and 3 are summarized together with the conduction velocity of the myocardial cell (circled number 1) and the average circumference length of the seeding surface (circled number 2) as listed in Table 2.

[Table 2]

| No. | Example | Used cell | Culture container | Stability of TW | Conduction velocity of myocardial cell (cm/s) (1) | Average circumference length (cm) (2) | (1)/(2) |
|---|---|---|---|---|---|---|---|
| 1 | Example 3 | Myocardial cell A | Culture container 1 | Up to Day 14 | 8 | 1.727 | 4.6 |
| 2 | Example 2 | Myocardial cell B | Culture container 1 | Up to Day 6 | 30 | 1.727 | 17.4 |
| 3 | Example 2 | Myocardial cell B | Culture container 2 | Up to Day 10 | 30 | 3.705 | 8.1 |
| 4 | Example 2 | Myocardial cell B | Culture container 3 | Up to Day 14 | 30 | 4.482 | 6.7 |
| 5 | Example 2 | Myocardial cell B | Culture container 4 | Up to Day 14 | 30 | 4.7 | 6.4 |

From Table 2 (in particular, results of No. 1 and No. 3), it can be presumed that a value obtained by dividing the circled number 1 by the circled number 2 (circled number 1/circled number 2) is needed to be about less than 10 for the TW to be stable up to Day 10 or more. In addition, in a case where the above-described relationship that circled number 1/circled number 2 is about less than 10 is satisfied, it can be presumed that the shape of the seeding surface is not limited as long as the shape is a closed loop shape.

Example 4: Production of myocardial cell layer (use of myocardial cell C)

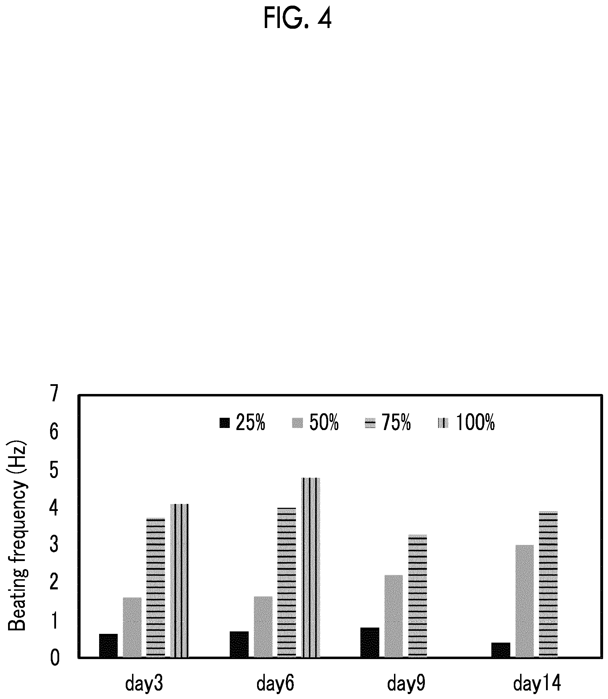

iCell (registered trademark) Cardiomyocytes[2] was thawed according to "iCell Cardiomyocytes[2] User's Guide" to prepare myocardial cells C (purity of the myocardial cells 100%). In addition, myocardium-derived fibroblasts (NHCF-V human cardiac fibroblasts, Lonza, CC-2904) were added to the myocardial cells C to prepare myocardial cells with a purity of 25%, 50%, and 75%, respectively. Each of the prepared myocardial cells was dispersed at $1 \times 10^6$ cells/ cm$^2$ and scattered on the seeding surface of the cells of the culture container 3 prepared in Example 1 to form a myocardial cell layer. The cells were cultured in the same manner as in Example 2, a beating frequency was measured, and stability of the TW was evaluated. The measurement results are shown in Fig. 4. The beating frequency in Day 6 was 0.7 Hz at a purity of 25% of the myocardial cell, 1.7 Hz at a purity of 50%, 4 Hz at a purity of 75%, and 4.8 Hz at a purity of 100%, and in a case where the myocardial cell purity was high, there was seen a tendency that the beating frequency was high. In addition, in the myocardial cells C having a purity of 100%, the TW was stable only up to Day 6 (beating frequency after Day 9 is not measured). Considering the fact that the purity of the myocardial cells B used in Example 2 is 50% to 90%, and the purity of the myocardial cells C used in Example 3 is 90%, it can be presumed that an appropriate purity of the myocardial cells is 50% to 90%.

Example 5: Rise in expression of myocardium-related genes by TW

A myocardial cell layer was formed using the myocardial cells B and the culture container 3 in the same manner as in Example 2. Two examples in which the generated TW was stably maintained up to Day 14 (TW-1, TW-2), and two examples in which the TW was stopped by exchanging the culture medium with a culture medium at 4°C on Day 2 (Control-1, Control-2), that is, a total of four examples were produced. The expression of each gene on Day 14 was examined. The gene expression was measured as follows.

Total RNA was purified using Trizol reagent (Life Technologies). The RNA concentration was measured using a spectrophotometer of Nanodrop 1000 (Thermo Fisher Scientific Inc.). cDNA was synthesized using Super Script 3 First-Strand Synthesis Super Mix for qRT-PCR (invitrogen), and cDNA sequence was measured using an Illumina Hiseq 4000 platform (Illumina). The results are shown in Table 3. In Table 3, adult means normal adult myocardial cells (BioChain: P1244122, USA). It was found that in a case where TW is maintained for a certain period of time, the myocardium-related genes in the cells constituting the myocardial cell layer rise and thus the value is closer to the adult.

[Table 3]

| Gene name | Control-1 | Control-2 | TW-1 | TW-2 | Adult |
|---|---|---|---|---|---|
| TNNI3 | 16.47 | 8.96 | 177.82 | 194.02 | 4443.55 |
| ITGA7 | 30.89 | 30.77 | 41.33 | 48.78 | 113.73 |
| IGF1 | 0.14 | 0.04 | 0.64 | 0.48 | 1.95 |
| SCN5A | 16.31 | 11.89 | 22.10 | 23.93 | 26.13 |
| KCNJ11 | 1.12 | 2.01 | 2.99 | 3.21 | 5.35 |
| Unit: FPKM | | | | | |

Example 6: Rise in expression of myocardial mature β-MHC marker by TW

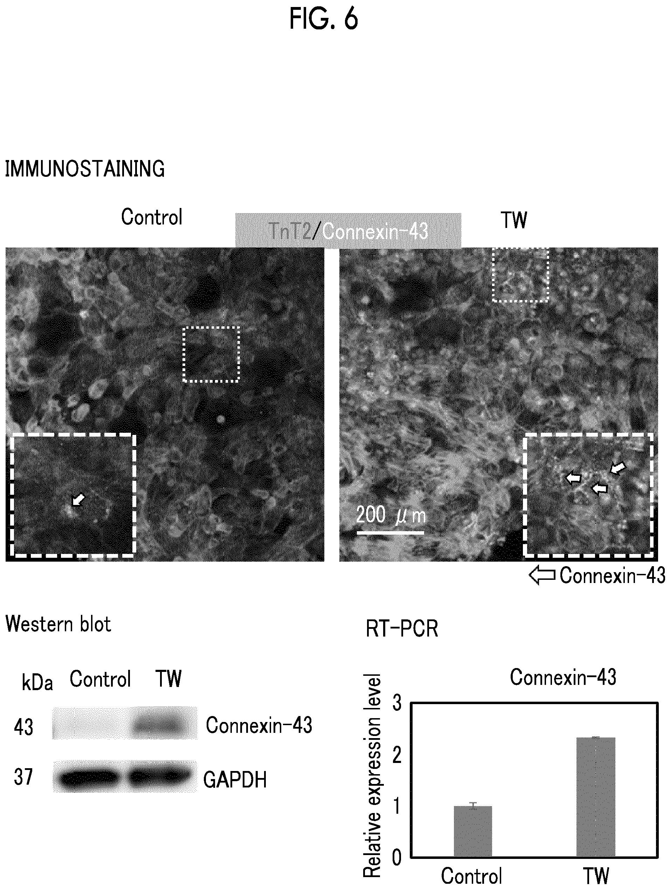

In the same manner as in Example 3, a myocardial cell layer was formed using the myocardial cells A and the culture container 1. Those in which the generated TW is stably maintained up to Day 14 (TW), and those in which the TW was stopped by exchanging the culture medium with a culture medium at 4°C on Day 2 (Control), that is, a total of two kinds were produced. On Day 14, the expression levels of a protein and a gene of β-MHC, which is a specific marker associated with myocardial cell maturation, were examined by the following methods. The results are shown in Fig. 6. The expression of β-MHC was clearly increased in TW as compared with Control.

Immunostaining:

Cells were fixed using 4% paraformaldehyde at room temperature for 30 minutes, and a cell membrane was permeated with 0.5% V/V Triton X-100 for 1 hour and incubated in a blocking solution for 16 hours. Thereafter, reaction was carried out using a primary antibody (β-MHC (Santa Cruz Biotechnology: SC-53089)) at 4°C for 16 hours. Reaction was carried out using a secondary antibody (the same as the primary antibody) at room temperature for 1 hour. The cell nucleus was stained using 300 nM DAPI (Wako) at room temperature for 30 minutes. During the transition of each step, washing was performed with PBS. The state of staining was imaged with a confocal microscope.

Western blot:

Protein was collected with SB10 lysis buffer (10% SDS, 5 mM EDTA, 50 mM Tris-HCl pH 7.4) (Sigma). Protein was quantified using a BCA protein assay kit (Thermo). The expression level of β-MHC was compared by a wet blot method. For the positive control, GAPDH was used.

RT-PCR:

Total RNA was purified using Trizol reagent (Life Technologies). The RNA concentration was measured using a spectrophotometer of Nanodrop 1000 (Thermo Fisher Scientific Inc.). cDNA was synthesized using Super Script 3 First-Strand Synthesis SuperMix for qRT-PCR (invitrogen). PCR was carried out using a StepOnePlus Real-Time PCR system (Life Technologies Corporation). As an adult, the gene expression of normal adult myocardial cells (BioChain: P1244122, USA) was also measured.

Example 7: Rise in expression of Connexin-43 by TW

In the same manner as in Example 3, a myocardial cell layer was formed using the myocardial cells A and the culture container 1. Those in which the generated TW was stably maintained up to Day 14 (TW), and those in which TW was stopped by exchanging the culture medium with a culture medium at 4°C on Day 2 (Control), that is, a total of two kinds were produced. The expression levels of the protein and gene of Connexin-43 on Day 14 were examined by the following methods. The results are shown in Fig. 6. The expression of Connexin-43 was clearly increased in TW as compared with Control.

Immunostaining:
The procedure was performed in the same manner as in Example 6, except that Connexin 43 (Santa Cruz: sc-9059) was used as the primary antibody and the secondary antibody.
Western blot:
The procedure was performed in the same manner as in Example 6.
RT-PCR:
The procedure was performed in the same manner as in Example 6 except that the gene expression of normal adult myocardial cells (BioChain: P1244122, USA) was not measured as adult.

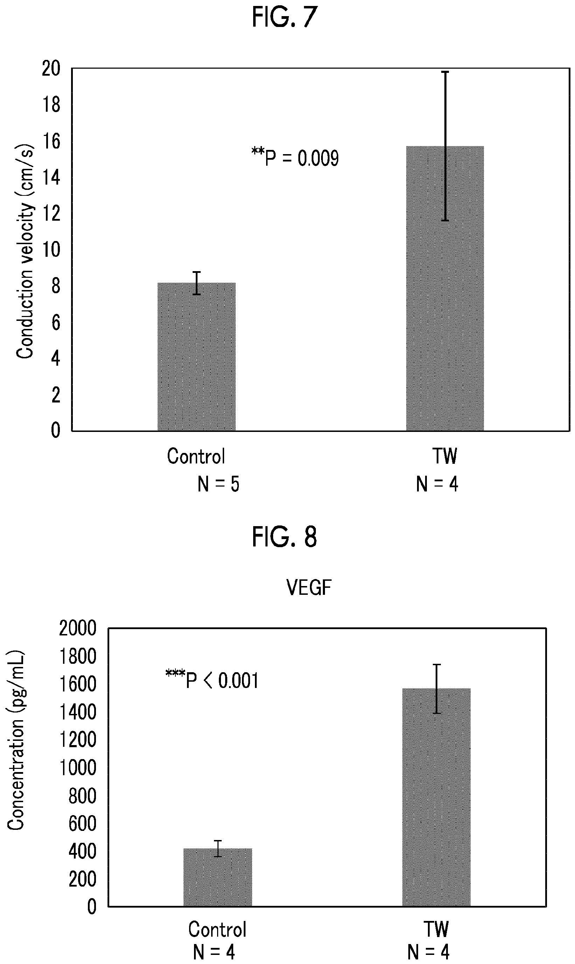

In addition, the conduction velocity of Control and TW was measured on Day 14. Regarding the TW, the conduction velocity was measured after the TW was stopped by exchanging the culture medium with a culture medium at 4°C on Day 14. Results are shown in Fig. 7.
It was confirmed that the conduction velocity between a cell and an adjacent cell is also increased in a case where the expression of Connexin-43, which is a marker of the Gap junction, is increased.

Example 8: An increase in secretion amount of angiogenesis secretion factor (VEGF) by TW and improvement of hypoxia resistance

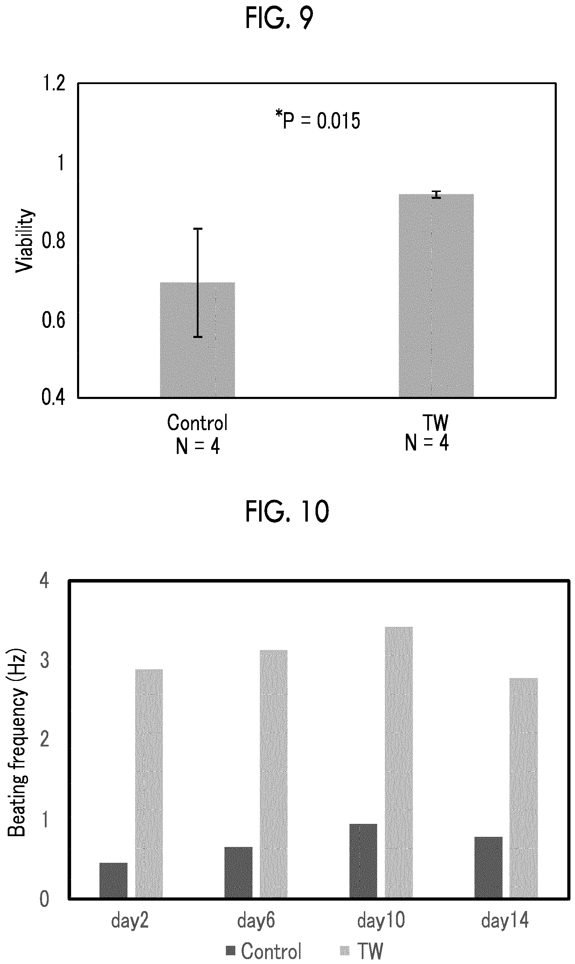

In the same manner as in Example 3, a myocardial cell layer was formed using the myocardial cells A and the culture container 1. Those in which the generated TW was stably maintained up to Day 14 (TW), and those in which TW was stopped by exchanging the culture medium with a culture medium at 4°C on Day 2 (Control), that is, a total of two kinds were produced. The culture supernatant was collected on Day 14, and the amount of angiogenesis secretion factor (VEGF) was measured by ELISA (ELISA Kit, R & D Systems, Inc.). The measurement results are shown in Fig. 8. The amount of VEGF was clearly increased in TW as compared with Control.
Similarly, two kinds of myocardial cell layers of TW and Control were produced and cultured in a 5% low oxygen environment for another 5 days after Day 14, and the viability of the cells was measured with a live/dead kit (Thermo Fisher Scientific Inc.). The general cell culture oxygen environment is 30%. The measurement results are shown in Fig. 9. In the case of TW, the viability after the culture in a low oxygen environment was improved as compared with Control. From the data, in a case where the myocardial cells in which the TW is maintained for a certain period of time are applied to the treatment of heart failure, it is expected that angiogenesis is promoted and the engraftment rate of the myocardial cells after transplantation is improved.

Example 9: Production of myocardial cell layer using scaffold material (use of myocardial cell B)

As a scaffold material, Upcell (registered trademark) (CellSeed, LLC) and a biodegradable fiber material (polylactic acid/glycolic acid copolymer (Sigma-Aldrich: PLGA, P1941)) having an orientation property were prepared and cut into the same shape and size as the bottom surface of the well of the 24-well plate used in Example 1, and each thereof was adhered to the wells of the 24-well plate with a silicone adhesive (one-pack condensation type RVT rubber (transparent) (Shin-Etsu Silicone)). A PDMS sheet 2 was created in the same manner as in Example 1 and adhered to the scaffold material. After being dried overnight, the resultant product was sterilized by UV irradiation for 30 minutes to create culture containers having a scaffold material (culture container 2 (Upcell), culture container 2 (biodegradable fiber material)).
Using the myocardial cells B, and the above-described culture container 2 (Upcell) and culture container 2 (biodegradable fiber material), a myocardial cell layer was formed in the same manner as in Example 2. It was confirmed on the second day (Day 2) that the TW was generated in the myocardial cell layer, using a microscope. In addition, it was confirmed that the TW was stable up to Day 14. It was confirmed that the myocardial cell layer according to the embodiment of the present invention can be applied to transplantation treatment to heart failure after maturation of myocardial cells on a scaffold material.

Example 10: Production of myocardial cell layer using scaffold material (use of myocardial cell C)

As a scaffold material, a biodegradable fiber material (polylactic acid/glycolic acid copolymer (PLGA)) having an orientation property was prepared, cut into the same shape and size as the bottom surface of the adhesive culture petri dish used in Example 1, and adhered to the adhesive culture petri dish with a silicone adhesive (one-pack condensation type RVT rubber (transparent) (Shin-Etsu Silicone)). A PDMS sheet 3 was created in the same manner as in Example 1 and adhered to the scaffold material. After being dried overnight, the resultant product was sterilized by UV irradiation for 30 minutes to create a culture container having a scaffold material (culture container 3 (biodegradable fiber material)).
Using the myocardial cell C (the myocardial cell with a purity of 80%, which was obtained in the same manner as in Example 4) and the above-described culture container 3 (biodegradable fiber material), a myocardial cell layer was formed in the same manner as in Example 4. It was confirmed on the second day (Day 2) that the TW was generated in the myocardial cell layer, using a microscope. In addition, the beating frequency was measured up to Day 14 (TW). As a control, the beating frequency was measured in the same manner for those (Control) in which the TW was stopped by exchanging the culture medium with a culture medium at 4°C on Day 2. The measurement results are shown in Fig. 10. In a case where the culture container 3 (biodegradable fiber material) was used, the beating frequency was stable at around 3 to 4 Hz up to Day 14.

Example 11: Production of myocardial cell layer using scaffold material (use of myocardial cell B)

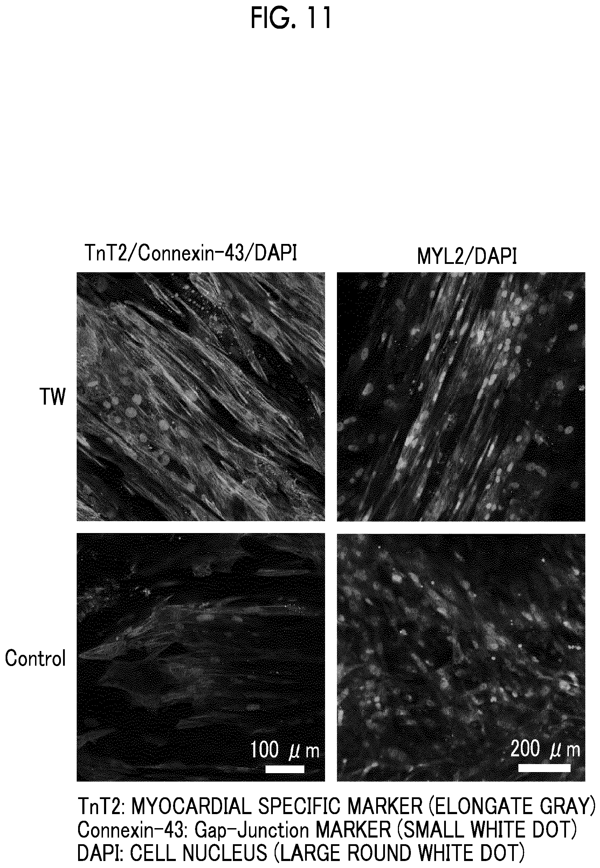

A culture container 3 (biodegradable fiber material) was created in the same manner as in Example 10. Using the myocardial cells B and the culture container 3 (biodegradable fiber material), a myocardial cell layer was formed in the same manner as in Example 2. Those in which the generated TW was stably maintained up to Day 14 (TW), and those in which TW was stopped by exchanging the culture medium with a culture medium at 4°C on Day 2 (Control), that is, a total of two kinds were produced. At the end of Day 14, immunostaining was performed by the method shown as follows. The results are shown in Fig. 11.
In the TW, the expression of the protein level of markers (TnnT2 and MYL2) associated with the specific maturity of the myocardial cell was increased as compared with Control. In addition, the TW sample had a mature myocardial structure having an orientation property along a direction of the fibers.

Immunostaining:

The procedure was performed in the same manner as in Example 6 except that as the primary antibody and the secondary antibody, cTnT (Santa Cruz Biotechnology: SC-20025) and Connexin 43 (Santa Cruz: sc-9059), or MYL2 (Proteintech: 10906-1-AP) was used.

Example 12: Relationship between production of myocardial cell layer using scaffold material (use of myocardial cell B) and electrical signal

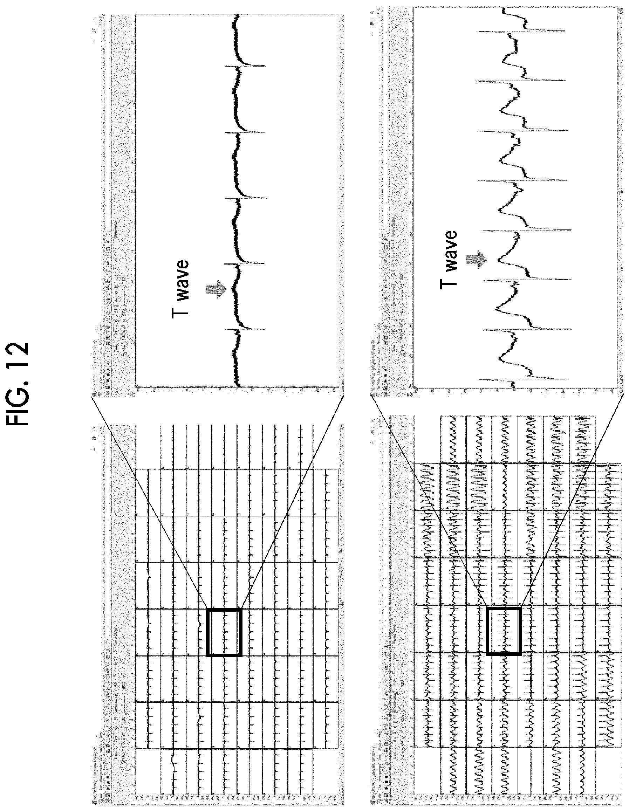

A culture container 3 (biodegradable fiber material) was created in the same manner as in Example 10. In addition, a culture container 3 (PC) was created in the same manner as for the culture container 3 (biodegradable fiber material), using polycarbonate (PC) porous membrane (Thermo Fisher Scientific, Inc., Nunc™ Polycarbonate Cell Culture Cell Inserts in Multi-Well Plates 140644) as a scaffold material. A myocardial cell layer was formed in the same manner as in Example 2, using the myocardial cells B, the culture container 3 (biodegradable fiber material), and the culture container 3 (PC). The generated TW was stably maintained up to Day 14. After Day 14, the electrical signal of myocardial spontaneous beating was measured by MEA by stopping the TW by exchanging the culture medium with a culture medium at 4°C. Specifically, data of extracellular Field Potentials (FPs) was recorded on a multielectrode array (MEA) system (USB-ME64-System, Multi Channel Systems, Germany).
The measurement results are shown in Fig. 12. It was possible to measure the electric signal regardless of the difference in the scaffold material. It was determined that the biodegradable fiber material has good contact properties with the MEA electrode chip, the amplitude of the T wave of the obtained electrical signal is large, and thus it is easy to detect the T wave. This feature is advantageous for measuring QT extension due to the addition of a drug in a case of being applied to drug evaluation.

Example 13: Production of myocardial cell layer using scaffold material (use of myocardial cell C), and change in QT interval and beating frequency by TW

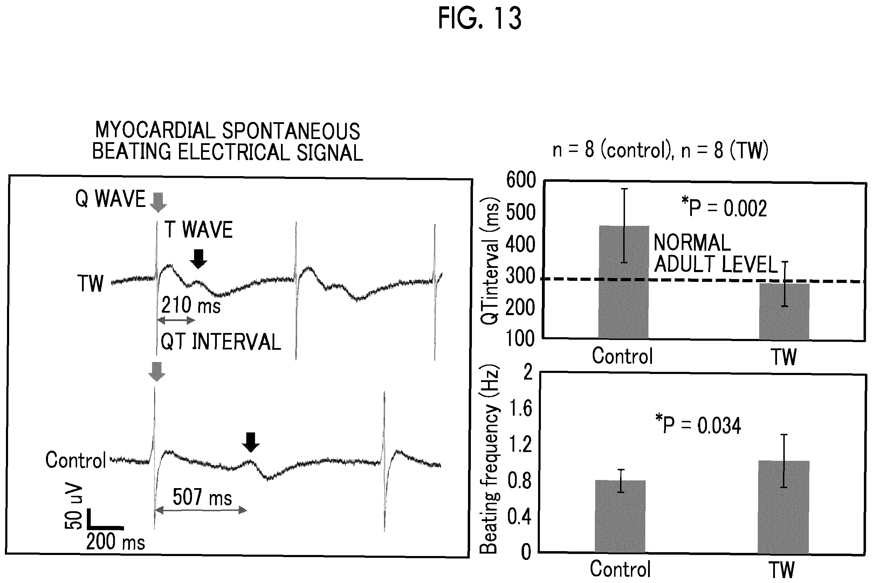

A culture container 3 (biodegradable fiber material) was created in the same manner as in Example 10. Using the myocardial cell C (the myocardial cell with a purity of 80%, which was obtained in the same manner as in Example 4) and the above-described culture container 3 (biodegradable fiber material), a myocardial cell layer was formed in the same manner as in Example 4. Those in which the generated TW was stably maintained up to Day 14 (TW), and those in which TW was stopped by exchanging the culture medium with a culture medium at 4°C on Day 2 (Control), that is, a total of two kinds were produced. After Day 14, the electrical signal of myocardial spontaneous beating was measured by MEA by stopping the TW by exchanging the culture medium with a culture medium at 4°C in the same manner as in Example 12.
The measurement results are shown in Fig. 13. In the graph, a normal adult level means normal adult myocardial cells (BioChain: P1244122, USA). In the sample in which the TW was stably maintained up to Day 14, the QT interval was close to the value of the normal adult. In addition, the beating frequency was improved. The beating frequency was measured in the same manner as in Example 2.

Example 14: Drug screening

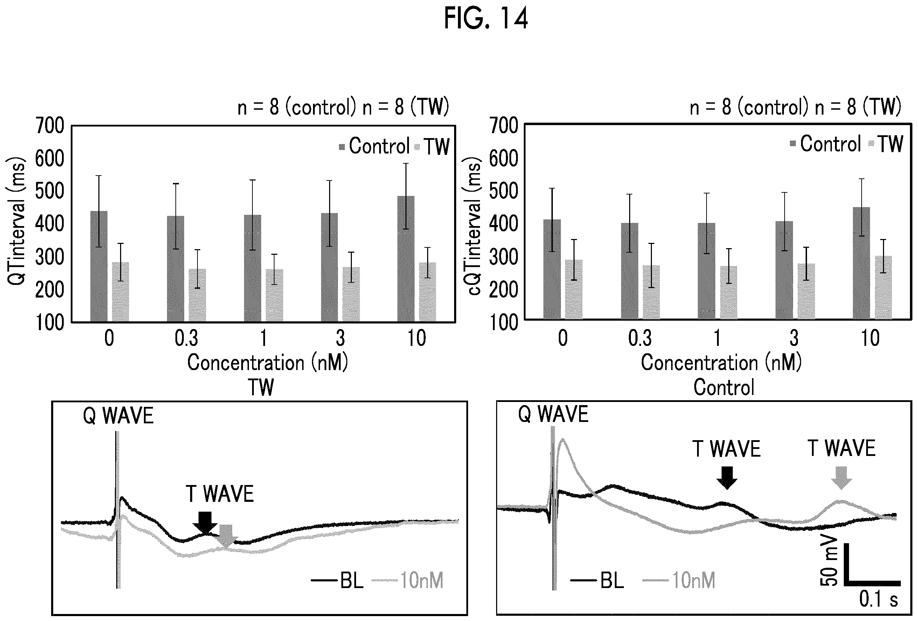

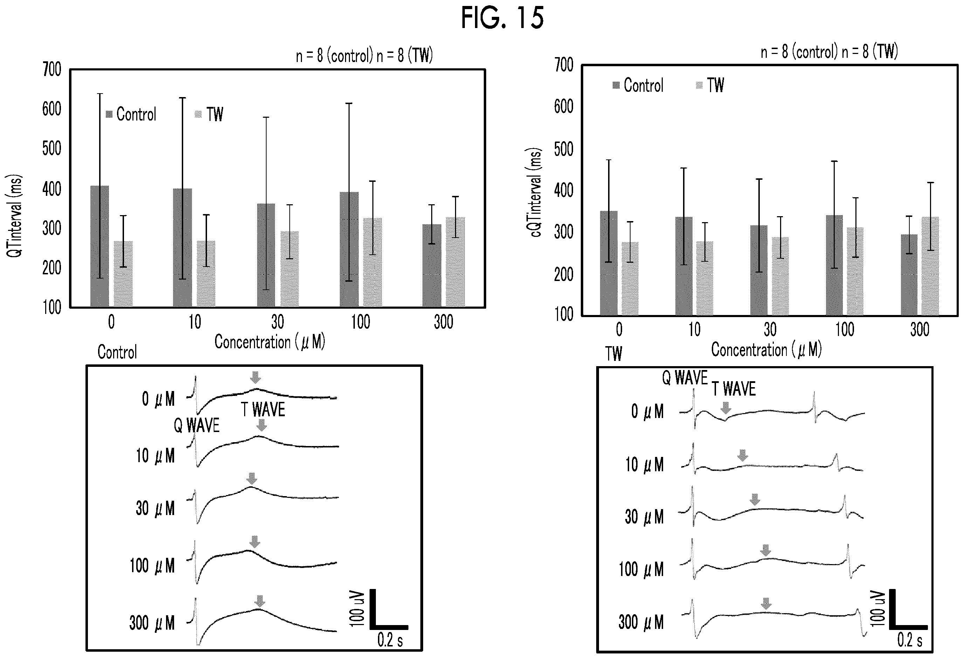

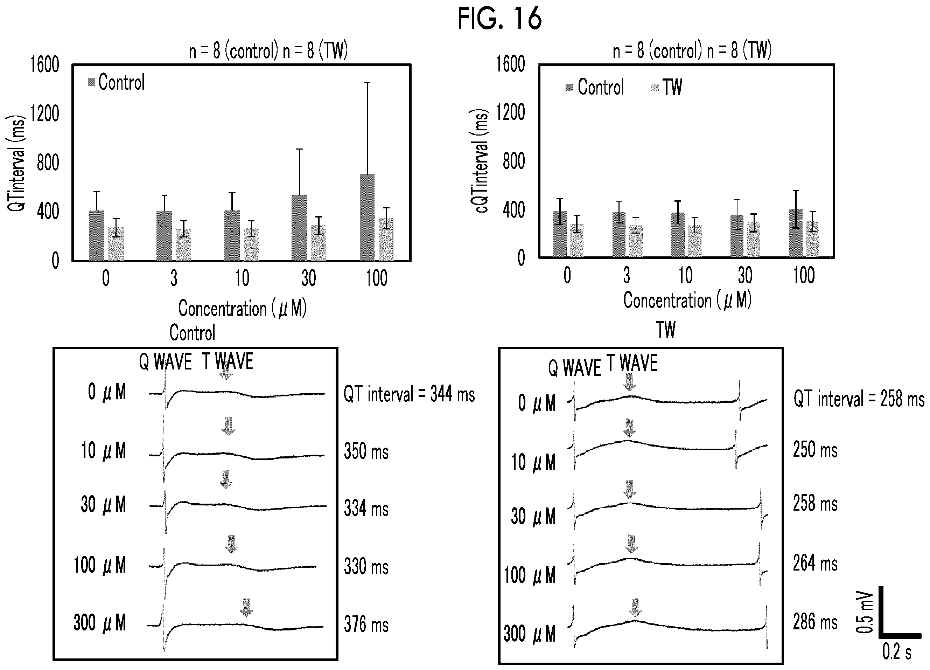

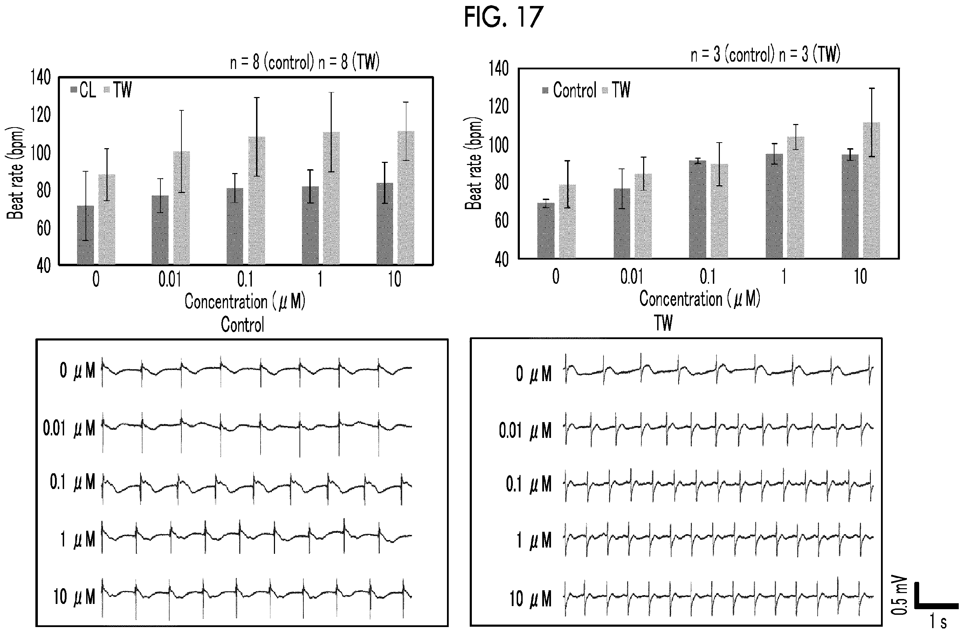

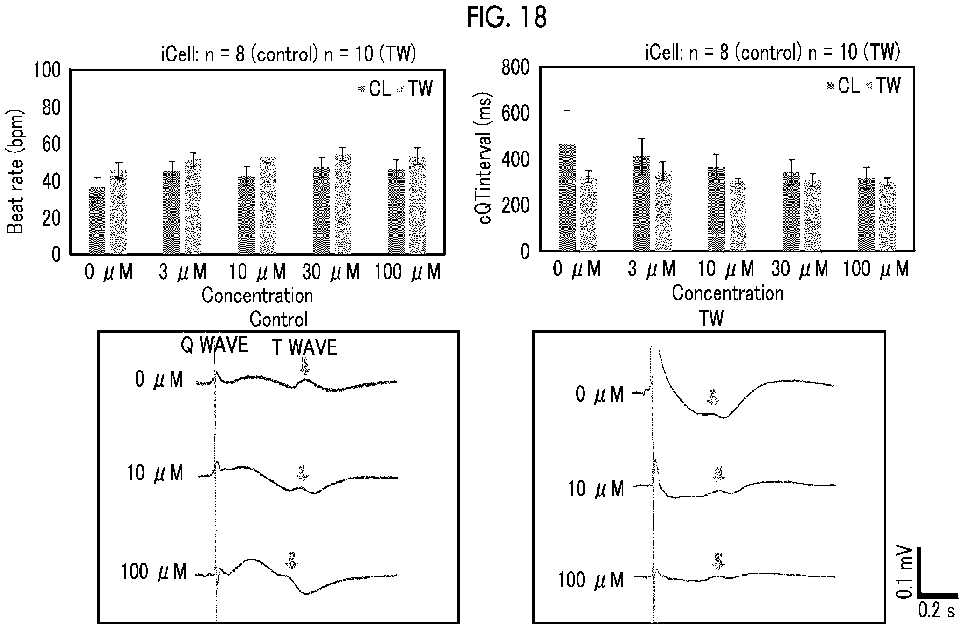

A myocardial cell layer was formed using the myocardial cell C (myocardial cell with a purity of 80%, which was obtained in the same manner as in Example 4) and the culture container 3 (biodegradable fiber material) in the same manner as in Example 13. Those in which the generated TW was stably maintained up to Day 14 (TW), and those in which TW was stopped by exchanging the culture medium with a culture medium at 4°C on Day 2 (Control), that is, a total of two kinds were produced. In the case of the TW, the TW was stopped by exchanging the culture medium with a culture medium at 4°C after Day 14, 1 ml of a culture medium containing various drugs (E-4031, ranolazine, mexiletine, isoproterenol, and aspirin (negative control)) at various concentrations was added to the myocardial cells, on Day 17, and 5 minutes later, the electrical signal of myocardial spontaneous beating was measured by MEA in the same manner as in Example 12. This data was analyzed using MC Rack (Multi Channel Systems, Inc.), the QT interval, or the beat rate was measured, and the cardiotoxicity was evaluated.
Results of evaluating E-4031 (potassium channel blocker) as a drug are shown in Fig. 14. Due to the addition of E-4031, the QT interval extension in both TW and Control was observed. The value of the QT interval extension of TW was smaller than Control.
Results of evaluating ranolazine (calcium channel blocker) as a drug are shown in Fig. 15. The values of Control had a large variation. The TW had a small variation, and exhibited stability and reproducibility between the samples.
Results of evaluating mexiletine (sodium channel blocker) as a drug are shown in Fig. 16. The variation in Control was large and the reproducibility was not good. The value of the QT interval extension of TW was smaller than Control.
Results of evaluating isoproterenol (β-adrenal receptor agonist) as a drug are shown in Fig. 17. In a case where isoproterenol was added, the myocardial beat rate was increased. The TW was more sensitive in reaction to isoproterenol as compared with Control.
Results of evaluating aspirin (negative control) as a drug are shown in Fig. 18. Regarding the QT interval extension and the myocardial beat rate, Control and TW were almost the same. As the concentration increased, the QT interval of Control was decreased.

Example 15: Drug screening

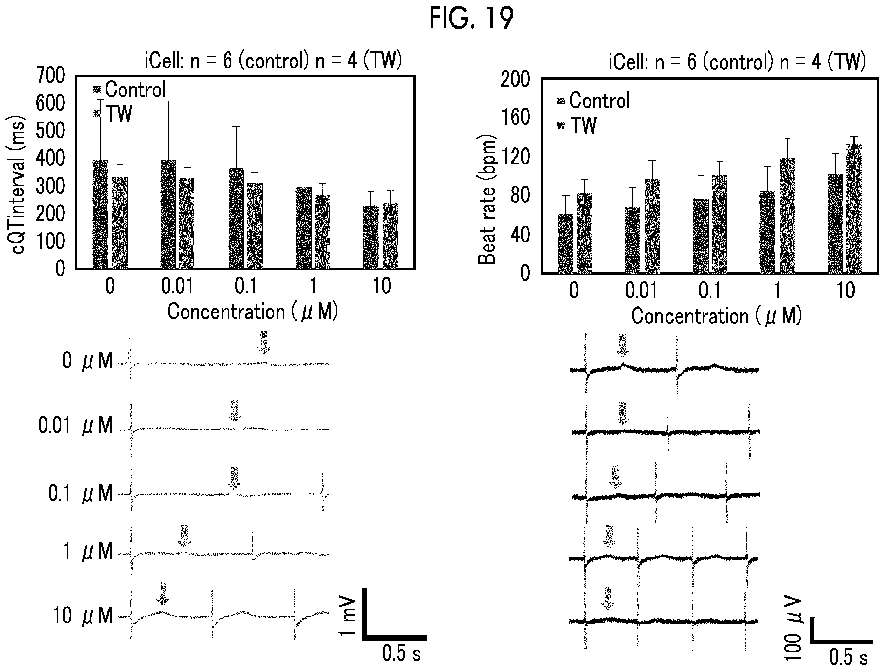

A drug (verapamil) was evaluated by the same procedure as in Example 14. The result is shown in Fig. 19. The addition of verapamil increased the myocardial beat rate. The TW was more sensitive in reaction to verapamil as compared with Control.

**Claims**

1. A production method of a myocardial cell layer, comprising the following steps (1) to (3),

   (1) seeding a myocardial cell induced from a pluripotent stem cell in a single layer on a seeding surface having a shape shown in the following (1-1) to form a myocardial cell layer,

   (1-1) in a case where spread of the seeding surface in a horizontal direction is a plane figure, a shape thereof is a shape obtained by hollowing out at least a part of a first plane figure of an optional shape having uniform spread in a second plane figure of another optional shape having uniform spread without contacting an outer periphery of the first plane figure of the optional shape, where an average value of a length of the outer periphery of the first plane figure and a length of an outer periphery of the second plane figure satisfies the following formula;

   $$x \geq v/10$$

   in the formula, x represents the average value of the length of the outer periphery of the first plane figure and the length of the outer periphery of the second plane figure, where a unit thereof is cm, and v represents a conduction velocity of the myocardial cell induced from the pluripotent stem cell, where a unit thereof is $cm \cdot s^{-1}$,

   (2) circulating and proceeding at least one electrical signal or a calcium signal in the myocardial cell layer,
   (3) culturing the cells for 8 days or more while maintaining a state of the (2).

2. The production method according to claim 1,
   wherein the pluripotent stem cell is an induced pluripotent stem cell or an embryonic pluripotent stem cell.

3. The production method according to claim 1 or 2,
   wherein an expression level of $\beta$-MHC in the myocardial cell layer is 10% or more of an expression level of a normal adult myocardial cell.

4. The production method according to any one of claims 1 to 3,
   wherein an expression level of Connexin-43 in the myocardial cell layer is 1.5 times or more an expression level of control cells which are not treated with a circulation wave.

5. The production method according to any one of claims 1 to 4,
   wherein a conduction velocity in the myocardial cell layer is 10 $cm \cdot s^{-1}$ or more.

6. The production method according to any one of claims 1 to 5,
   wherein a VEGF secretion amount from the myocardial cell layer is 2 times or more a VEGF secretion amount from control cells which are not treated with a circulation wave.

7. The production method according to any one of claims 1 to 6,
   wherein hypoxia resistance of the myocardial cell layer is improved as compared with control cells which are not treated with a circulation wave.

8. The production method according to any one of claims 1 to 7,

wherein the first plane figure is a circle, and the second plane figure is a circle having a diameter smaller than a diameter of the circle of the first plane figure.

9. The production method according to any one of claims 1 to 7,

wherein the first plane figure is a shape obtained by cutting a substantial rectangle from a circle to be in contact with an outer periphery of the circle, and the second plane figure is a shape of a Landolt ring, and
at least the part of the first plane figure is hollowed out in the second plane figure such that the rectangle is inserted between cuts of the Landolt ring.

10. The production method according to any one of claims 1 to 7,
wherein the first plane figure is a recessed type, and the second plane figure is a recessed type smaller than the first plane figure.

11. The production method according to any one of claims 1 to 10,
wherein an expression level of one or more myocardial maturation markers selected from TNNI3, ITGA7, IGF1, SCN5A, and KCNJ11 in the myocardial cell layer is 1.3 times or more an expression level in control cells which are not treated with a circulation wave.

12. The production method according to any one of claims 1 to 11, further comprising:
a step of performing co-culture with a fibroblast.

13. The production method according to any one of claims 1 to 12,
wherein the myocardial cell layer is formed on a biodegradable or non-biodegradable scaffold.

14. The production method according to any one of claims 1 to 13,
wherein a seeding density of the myocardial cells in the step (1) is $2 \times 10^5$ to $1 \times 10^6$ cells/cm$^2$.

15. The production method according to any one of claims 1 to 14,
wherein the pluripotent stem cell is a pluripotent stem cell having a gene mutation associated with a heart disease.

16. A myocardial cell layer obtained by the production method according to any one of claims 1 to 15.

17. A myocardial cell layer having the following characteristics (1) to (5),

(1) the myocardial cell layer includes a myocardial cell induced from a pluripotent stem cell,
(2) the myocardial cell layer is a single layer,
(3) the myocardial cell layer satisfies at least one of the following (3-1) to (3-8),

(3-1) an expression level of β-MHC in the myocardial cell layer is 10% or more of a normal adult myocardial cell;
(3-2) a VEGF secretion amount from the myocardial cell layer is 2 times or more that of control cells which are not treated with a circulation wave;
(3-3) an expression level of Connexin-43 in the myocardial cell layer is 1.5 times or more that of the control cells which are not treated with the circulation wave;
(3-4) an expression level of TNNI3 in the myocardial cell layer is 2 times or more that of the control cells which are not treated with the circulation wave;
(3-5) an expression level of ITGA7 in the myocardial cell layer is 1.2 times or more that of the control cells which are not treated with the circulation wave;
(3-6) an expression level of IGF1 in the myocardial cell layer is 1.5 times or more that of the control cells which are not treated with the circulation wave;
(3-7) an expression level of SCN5A in the myocardial cell layer is 1.2 times or more that of control cells which are not treated with the circulation wave;
(3-8) an expression level of KCNJ11 in the myocardial cell layer is 1.3 times or more that of control cells which are not treated with the circulation wave;

(4) in a case where spread of a seeding surface in a horizontal direction is a plane figure, the shape thereof is a shape obtained by hollowing out at least a part of a first plane figure of an optional shape having uniform spread in a second plane figure of another optional shape having uniform spread without contacting an outer periphery of the first plane figure of the optional shape,

(5) an average value of a length of the outer periphery of the first plane figure and a length of an outer periphery of the second plane figure satisfies the following formula,

$$x \geq v/10$$

in the formula, x represents the average value of the length of the outer periphery of the first plane figure and the length of the outer periphery of the second plane figure, where a unit thereof is cm, and v represents a conduction velocity of the myocardial cell induced from the pluripotent stem cell, where a unit thereof is $cm \cdot s^{-1}$.

**18.** A kit for evaluating a pharmaceutical candidate substance, comprising:
the myocardial cell layer according to claim 16 or 17.

**19.** A transplantation material comprising:
the myocardial cell layer according to claim 16 or 17.

**20.** An evaluation method of a pharmaceutical candidate substance, comprising:

(1) bringing the myocardial cell layer according to claim 16 or 17 into contact with a candidate substance;
(2) detecting an electrical signal or a calcium signal of the myocardial cell layer in contact with the candidate substance; and
(3) comparing the electrical signal or the calcium signal, which is detected in the step (2), with a control electrical signal or a control calcium signal.

# FIG. 1

PDMS SHEET 1

PDMS SHEET 2

PDMS SHEET 3

PDMS SHEET 4

EP 4 424 819 A1

# FIG. 2

CULTURE CONTAINER 1

CULTURE CONTAINER 2

CULTURE CONTAINER 3

CULTURE CONTAINER 4

15.6 mm

SEEDING SURFACE OF CELL

DOTTED LINE SHOWING INNER FRAME OF WELL

PDMS SHEET

DOTTED LINE SHOWING INNER FRAME OF PETRI DISH

# FIG. 3

CULTURE CONTAINER 1

CULTURE CONTAINER 2

CULTURE CONTAINER 3

CULTURE CONTAINER 4

EP 4 424 819 A1

FIG. 4

# FIG. 5

IMMUNOSTAINING

Control      β-MHC/DAPI      TW

50 μm

Western blot

kDa Control    TW

150                    β-MHC

37                     GAPDH

RT-PCR

# FIG. 6

IMMUNOSTAINING

Control  TnT2/Connexin-43  TW

200 μm

⇐ Connexin-43

Western blot

kDa Control TW

43  Connexin-43

37  GAPDH

RT-PCR

Connexin-43

Relative expression level

## FIG. 7

**P = 0.009

Control
N = 5

TW
N = 4

## FIG. 8

VEGF

***P < 0.001

Control
N = 4

TW
N = 4

## FIG. 9

## FIG. 10

# FIG. 11

TnT2/Connexin-43/DAPI      MYL2/DAPI

TnT2: MYOCARDIAL SPECIFIC MARKER (ELONGATE GRAY)
Connexin-43: Gap-Junction MARKER (SMALL WHITE DOT)
DAPI: CELL NUCLEUS (LARGE ROUND WHITE DOT)

# FIG. 12

EP 4 424 819 A1

## FIG. 13

MYOCARDIAL SPONTANEOUS
BEATING ELECTRICAL SIGNAL

Q WAVE

T WAVE

TW

210 ms

QT INTERVAL

Control

507 ms

50 uV

200 ms

n = 8 (control), n = 8 (TW)

*P = 0.002

NORMAL ADULT LEVEL

QT interval (ms)

Control          TW

*P = 0.034

Beating frequency (Hz)

Control          TW

EP 4 424 819 A1

## FIG. 14

n = 8 (control) n = 8 (TW)

n = 8 (control) n = 8 (TW)

■ Control ■ TW

■ Control ■ TW

QT interval (ms)

cQT interval (ms)

Concentration (nM)

Concentration (nM)

TW

Control

Q WAVE

Q WAVE

T WAVE

T WAVE

T WAVE

—BL —10nM

—BL —10nM

50 mV

0.1 s

FIG. 15

FIG. 16

FIG. 17

EP 4 424 819 A1

# FIG. 18

iCell: n = 8 (control) n = 10 (TW)

Beat rate (bpm) vs Concentration

■CL ■TW

0 $\mu$M  3 $\mu$M  10 $\mu$M  30 $\mu$M  100 $\mu$M

iCell: n = 8 (control) n = 10 (TW)

cQTinterval (ms) vs Concentration

■CL ■TW

0 $\mu$M  3 $\mu$M  10 $\mu$M  30 $\mu$M  100 $\mu$M

Control

Q WAVE    T WAVE

0 $\mu$M

10 $\mu$M

100 $\mu$M

TW

0 $\mu$M

10 $\mu$M

100 $\mu$M

0.1 mV

0.2 s

EP 4 424 819 A1

FIG. 19

## INTERNATIONAL SEARCH REPORT

| International application No. |
|---|
| **PCT/JP2022/040098** |

### A. CLASSIFICATION OF SUBJECT MATTER

*C12N 5/077*(2010.01)i; *A61L 27/38*(2006.01)i; *A61P 9/04*(2006.01)i; *C12N 5/0735*(2010.01)i; *C12Q 1/02*(2006.01)i
FI:   C12N5/077; C12N5/0735; A61L27/38 300; A61L27/38 120; C12Q1/02; A61P9/04

According to International Patent Classification (IPC) or to both national classification and IPC

### B. FIELDS SEARCHED

Minimum documentation searched (classification system followed by classification symbols)

C12N5/077; A61L27/38; A61P9/04; C12N5/0735; C12Q1/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | JP 2016-41074 A (TOKYO MEDICAL & DENTAL UNIVERSITY) 31 March 2016 (2016-03-31)<br>claims, paragraphs [0037], [0049], [0066]-[0068], fig. 11, 12, 23, 24 | 1-20 |
| A | WO 2008/149976 A1 (NATIONAL UNIVERSITY CORPORATION TOKYO MEDICAL AND DENTAL UNIVERSITY) 11 December 2008 (2008-12-11)<br>claims, paragraphs [0012], [0025], [0035], fig. 5-10 | 1-20 |
| A | WO 2018/124210 A1 (KYOTO UNIVERSITY) 05 July 2018 (2018-07-05)<br>claims | 1-20 |
| A | US 2013/0196435 A1 (THE REGENTS OF THE UNIVERSITY CALIFORNIA) 01 August 2013 (2013-08-01)<br>claims, paragraphs [0037], [0038], fig. 14 | 1-20 |
| A | JP 2006-94703 A (ON-CHIP CELLOMICS CONSORTIUM CO., LTD.) 13 April 2006 (2006-04-13)<br>claims, fig. 3 | 1-20 |

☑ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 December 2022** | **20 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

# EP 4 424 819 A1

## INTERNATIONAL SEARCH REPORT

International application No.

**PCT/JP2022/040098**

### C. DOCUMENTS CONSIDERED TO BE RELEVANT

| Category* | Citation of document, with indication, where appropriate, of the relevant passages | Relevant to claim No. |
|---|---|---|
| A | WO 2013/061849 A1 (NATIONAL UNIVERSITY CORPORATION TOKYO MEDICAL AND DENTAL UNIVERSITY) 02 May 2013 (2013-05-02) | 1-20 |
| A | JP 2016-504022 A (THE GOVERNING COUNCIL OF THE UNIVERSITY OF TORONTO) 12 February 2016 (2016-02-12) | 1-20 |
| A | ZLOCHIVER, S. et al. Electrotonic Myofibroblast-to-Myocyte Coupling increases Propensity to Reentrant Arrhythmias in Two-Dimensional Cardiac Monolayers, Biophysical Journal, 2008, vol. 95, pp. 4469-4480 abstract | 1-20 |

Form PCT/ISA/210 (second sheet) (January 2015)

43

**INTERNATIONAL SEARCH REPORT**
Information on patent family members

International application No.

**PCT/JP2022/040098**

| Patent document cited in search report | | | Publication date (day/month/year) | Patent family member(s) | | | Publication date (day/month/year) |
|---|---|---|---|---|---|---|---|
| JP | 2016-41074 | A | 31 March 2016 | US | 2013/0230881 | A1 | |
| | | | | claims, examples | | | |
| | | | | WO | 2012/043820 | A1 | |
| | | | | EP | 2626411 | A1 | |
| WO | 2008/149976 | A1 | 11 December 2008 | US | 2010/0173351 | A1 | |
| | | | | claims, examples | | | |
| | | | | EP | 2157165 | A1 | |
| | | | | CN | 101730736 | A | |
| WO | 2018/124210 | A1 | 05 July 2018 | (Family: none) | | | |
| US | 2013/0196435 | A1 | 01 August 2013 | (Family: none) | | | |
| JP | 2006-94703 | A | 13 April 2006 | US | 2009/0042200 | A1 | |
| | | | | claims | | | |
| | | | | EP | 1626278 | A2 | |
| WO | 2013/061849 | A1 | 02 May 2013 | US | 2014/0349332 | A1 | |
| | | | | EP | 2772531 | A1 | |
| JP | 2016-504022 | A | 12 February 2016 | US | 2015/0313704 | A1 | |
| | | | | WO | 2014/085933 | A1 | |
| | | | | EP | 2929014 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018124210 A
- WO 2013111875A A
- WO 2015182765 A

- WO 2020027278 A
- WO 2012026491 A

### Non-patent literature cited in the description

- *Nature,* 2018, vol. 556, 239-243
- *Nature Methods,* 2013, vol. 10, 781-787
- *Nature Communications,* 2020, vol. 11
- *Scientific Reports,* 2020, vol. 10
- *Circulation.,* 09 May 2017, vol. 135 (19), 1832-1847
- *Scientific Reports,* 2014, vol. 4
- *Communications Biology,* 2020, vol. 3

- **MINAMI I et al.** *Cell Rep.,* 29 November 2012, vol. 2 (5), 1448-60
- *CHEMICAL ABSTRACTS,* 179248-59-0
- *CHEMICAL ABSTRACTS,* 733009-42-2
- *CHEMICAL ABSTRACTS,* 179248-61-4
- *CHEMICAL ABSTRACTS,* 879127-07-8
- *CHEMICAL ABSTRACTS,* 63177-57-1